# EUROPEAN PATENT APPLICATION

(11) **EP 1 416 412 A2**
(43) Date of publication of application: **06.05.2004**
(21) Application number: 03256583.0
(22) Date of filing: 20.10.2003
(51) Int. Cl.: G06F 17/60, G06F 19/00

(54) **Medical image administration system and method**

(30) Priority: 31.10.2002 JP 2002317243; 26.03.2003 JP 2003084708; 26.03.2003 JP 2003084753; 28.03.2003 JP 2003091600
(71) Applicant: KONICA MINOLTA HOLDINGS, INC., Tokyo 100-0005 (JP)
(72) Inventor: Moriyama, Naoto, Hachioji-shi Tokyo 192-8505 (JP); Motoki, Wataru, Hachioji-shi Tokyo 192-8505 (JP); Shiibashi, Takao, Hachioji-shi Tokyo 192-8505 (JP); Umeki, Mamoru, Hachioji-shi Tokyo 192-8505 (JP)
(74) Representative: Rees, Alexander Ellison

(57) **Abstract**

A medical image photographing system includes : a medical image generating apparatus for photographing a patient to generate image data of the patient; an information management apparatus for generating photographing order information including patient identification information of the patient to be photographed and/or a photographing condition for the patient, the information management apparatus being allowed to transmit the generated photographing order information through a network to another apparatus; one or more control apparatuses that are connectable to the information management apparatus through the network to obtain the generated photographing order information; and one or more portable terminals that are connectable to at least one of the control apparatuses to obtain the photographing order information from the control apparatus connected thereto and display the obtained photographing order information.

## Description

### BACKGROUND OF THE INVENTION

### Field of the Invention

The present invention relates to medical image photographing system and apparatus for achieving medical images such as radiographing images or the like.

### Description of the Related Art

Digitalization of medical images achieved by photographing a patient has been implemented in a medical field, and there has been utilized such a medical image photographing system that digital image data are achieved by using a computed radiographing apparatus (hereinafter referred to as "CR" (Computed Radiography)), a magnetic resonance imaging apparatus (hereinafter referred to as "MRI" (Magnetic Resonance Imaging)) or the like, and then the digital image data thus achieved are distributed to an image management apparatus or a display device through a communication network (see JP-A-2000-139888, for example).

Of these image photographing apparatuses, CR uses a radioactive phosphor panel (hereinafter referred to as "imaging plate") having a photostimulable phosphor formed on a support member thereof. According to CR, radiation ray transmitted through a patient is irradiated to the imaging plate, so that the radiation energy of the radiation ray is absorbed by the imaging plate. Thereafter, excitation light is irradiated to the imaging plate to excite the radiation energy absorbed by the imaging plate and radiate fluorescence. The fluorescence thus radiated is photoelectrically converted to an image signal.

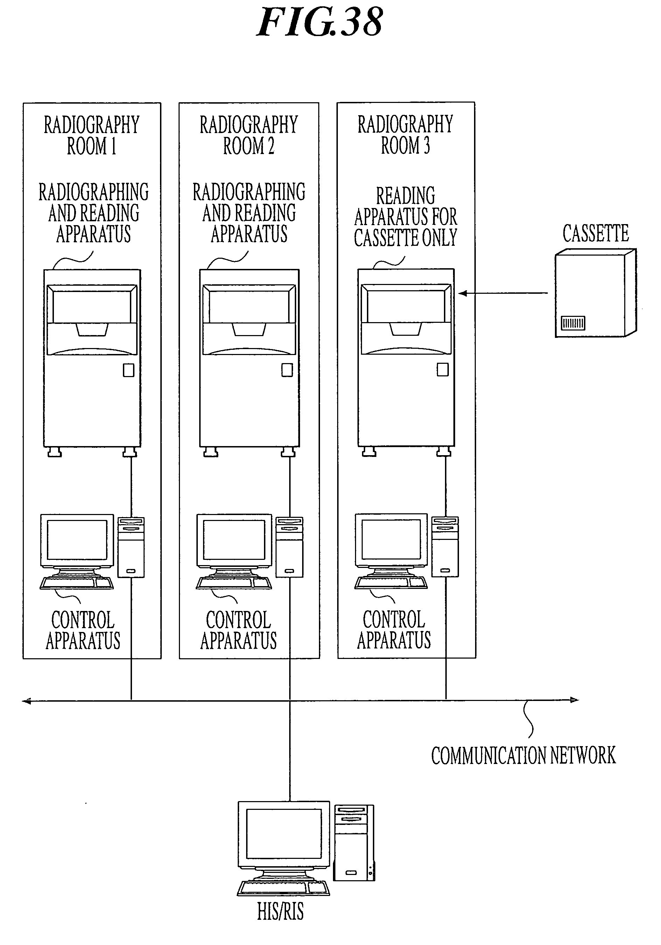

The medical image photographing system as described above will be described with reference to FIG. 38.

According to the conventional medical image photographing system, it is general that a patient is fed to a radiographing room and subjected to radiograph therein. In each radiographing room are fixedly set up a radiographing reading apparatus containing a phosphor plate for carrying out a radiographing and image-reading operation, a cassette-dedicated reading apparatus for reading out an image from a portable cassette in which a phosphor plate is mounted, etc.

A radiography operator carries out X-ray photographing on the patient on the basis of radiographing order information based on HIS (Hospital Information System) or RIS (Radiology Information System) for managing diagnosis information in a hospital or radiology by using a radiographing apparatus set up in a radiographing room of the hospital in advance. Here, the content of the radiographing order information contains the name of a patient, patient ID information, sex, age, hospital room, department in attendance, the name of a doctor in attendance, radiographing conditions, etc.

In the meantime, particularly, a portable radiographing apparatus (hereinafter referred to as "medical radiographing apparatus") which enables X-ray photographing in a hospital room where a patient is accommodated has been recently implemented. If the medical photographing apparatus described above is used, it would be possible to easily carry out X-ray photographing on even a patient who is difficult in walking (movement) due to severe injury and thus unmovable to a radiographing room. Furthermore, an image information reading apparatus for reading out radiographs from a cassette is set up at predeterminedplaces, each nurse station or the like on each floor of a hospital ward. By using this image information reading apparatus, the operator can readily check the radiography result without moving on the floor or between floors.

Here, the cassette has an imaging plate as described above. Each cassette or imaging plate is marked with an inherent identification number, so that the cassette or imaging plate can be identified on the basis of the identification number.

Furthermore, there has been developed a technique in which information achieved from a predetermined data base through a portable terminal such as PDA (Personal Digital assistance) or the like is added to another information to achieve composite information and then the composite information thus achieved is uploaded to the original data base (see JP-A-10-275163, for example).

The conventional techniques described above have the following problems. When radiography is carried out in a hospital room by using a portable radiographing apparatus as described above, a radiography operator fills an order content for plural items of photographic order information and the number of a cassette to be used for the radiographing order concerned in a predetermined order sheet every patient and radiographing condition in advance, carries the order sheet concerned (and the cassette marked with the number concerned) to a hospital room in which a patient is accommodated, carries out X-ray photographing using the portable radiographing apparatus while checking the order sheet, and then reads out radiographs achieved through the radiographing operation by using an image information reading apparatus at a place in the hospital ward.

Therefore, the operator must fill the order content for plural items in plural order sheets every patient and radiographing condition each time, and this makes the processing connected with the X-ray photographing cumbersome and also induces such an unanticipated situation as erroneous fill-in or the like. Furthermore, there may occur such an unanticipated situation that the order sheets are torn or lost while the operator moves in or out of the radiographing room or comes and goes among plural hospital rooms because the order sheets are formed of paper.

Furthermore, in the conventional system architecture, the same radiographing order information is redundantly distributed from HIS or RIS to all the control apparatuses, so that plural radiography operators may check the same radiographing order information by using different control apparatuses and make preparations for radiography for the same patient at the same time, so that a needless preparation work is redundantly carried out. Besides, when radiography is erroneously redundantly carried out on the patient, the patient is exposed to needless radiation.

### SUMMARY OF THE INVENTION

A first object of the present invention is to provide an efficient patient photographing work flow in a hospital.

A second object of the present invention is to enable photographing at a doctor's round visit place to be carried out efficiently.

A third object of the present invention is to readily expand the functions of an existing photographing system in low cost.

In accordance with the first aspect of the invention, a medical image photographing system comprises:
a medical image generating apparatus for photographing a patient to generate image data of the patient;
an information management apparatus for generating photographing order information including patient identification information of the patient to be photographed and/or a photographing condition for the patient, the information management apparatus being allowed to transmit the generated photographing order information through a network to another apparatus;
one or more control apparatuses that are connectable to the information management apparatus through the network to obtain the generated photographing order information; and
one or more portable terminals that are connectable to at least one of the control apparatuses to obtain the photographing order information from the control apparatus connected thereto and display the obtained photographing order information.

Preferably, the medical image photographing system further comprises a plurality of control apparatuses,
wherein each of the one or more portable terminals is connectable to all of the plurality of control apparatuses through the network.

Preferably, the medical image generating apparatus uses an image recording panel whenever the patient is photographed;
the one or more portable terminals obtain recording panel identification information of each image recording panel, and store the photographing order information and the obtained recording panel identification information so as to set a correspondence of the photographing order information to the obtained recording panel identification information;
the one or more portable terminals transmits the stored photographing order information and the stored recording panel identification information to one of the plurality of control apparatuses;
the one of the plurality of control apparatuses transmits the photographing order information and the recording panel identification information received fromthe one or more portable terminals, to the information management apparatus; and
the information management apparatus stores the recording panel identification information so as to correspond to the photographing order information.

Preferably, the medical image photographing system further comprises an image information reading apparatus for reading out a photographing image from the image recording panel,
wherein the image information reading apparatus reads out the recording panel identification information of the image information recording panel, obtains relating information of the photographing orderinformation,whichincludesa processing condition through the control apparatus, and reads out the image under the obtained processing condition.

Preferably, the medical image photographing system further comprises a plurality of portable terminals,
wherein the information management apparatus stores identification information of a first portable terminal of the plurality portable terminals for reading out the photographing order information, so as to correspond to the read out photographing order information; and when the photographing order information is read out by the first portable terminal of the plurality of portable terminals and then a command for reading out the photographing order information is output from a second portable terminal which is different from the first portable terminal of the plurality of portable terminals, the second portable terminal of the plurality of portable terminals is notified that the photographing order information has been already read out on the basis of the stored information.

In accordance with the second aspect of the invention, a medical image photographing method in a medical image photographing system comprising an information management apparatus, one or more control apparatuses and one or more portable terminals, comprises:
photographing a patient to generate image data of the patient;
generating photographing order information including patient identification information of the patient to be photographed and/or a photographing condition for the patient;
transmitting the generated photographing order information through a network to another apparatus;
obtaining the transmitted photographing order information by transmitting the transmitted photographing order information from the control apparatus to the portable terminal;
display the obtained photographing order information.

Preferably, in the above method, the medical image photographing system further comprises a plurality of control apparatuses,
wherein the medical image photographing method further comprises:
using an image recording panel whenever the patient is photographed;
obtaining recording panel identification information of each image recording panel;
storing the photographing order information and the obtained recording panel identification information so as to set a correspondence of the photographing order information to the obtained recording panel identification information;
transmitting the stored photographing order information and the stored recording panel identification information to one of the plurality of control apparatuses;
transmitting the photographing order information and the recording panel identification information received from the one or more portable terminals, to the information management apparatus; and
storing the recording panel identification information in the information management apparatus so as to correspond to the photographing order information.

Preferably, the method further comprises:
obtains relating information of the photographing order information, which includes a processing condition through the control apparatus, and
reading out the image under the obtained processing condition.

Preferably, in the above method, the medical image photographing system further comprises a plurality of portable terminals; and
wherein the medical image photographing method further comprises:
storing identification information of a first portable terminal of the plurality portable terminals for reading out the photographing order information, so as to correspond to the read out photographing order information;
wherein when the photographing order information is read out by the first portable terminal of the plurality of portable terminals and then a command for reading out the photographing order information is output from a secondportable terminal which is different from the first portable terminal of the plurality of portable terminals, the second portable terminal of the plurality of portable terminals is notified that the photographing order information has been already read out on the basis of the stored information.

According to the system or method described above, it is unnecessary to individually fill the order contents for plural items on plural order sheets every patient and every photographing condition, and the processing needed for X-ray photographing can be facilitated. In addition, there can be avoided such an unanticipated situation that erroneous data are filled in an order sheet, and also there can be avoided such an unanticipated situation that an order sheet is torn or lost while an operator repetitively moves in or out of a photographing room or comes and goes among plural hospital rooms.

Furthermore, when at least one control apparatus is set up on each floor in a hospital ward, it is unnecessary for the operator to move on a floor or between floors in order to look for the control apparatus and achieve photographing order information, and the operator can easily achieve the photographing order information from any control apparatus set up on the same floor as the hospital room of the patient to be subjected to radiography. In this case, The photographing order information containing the panel identification information inherent to a photographing panel can be easily uploaded from any control apparatus to an information management apparatus.

That is, the medical photographing operation in the hospital room and the image processing on the photographs (photographic image data) achieved through the photographing operation can be easily and efficiently performed.

Preferably, the medical image photographing system further comprises a plurality of portable terminals,
wherein the photographing order information, transmitted from one of the plurality of control apparatuses to one of the plurality of portable terminals, is prohibited from being transmitted from the control apparatus to another portable terminal.

Preferably, in the above method, the medical image photographing system further comprises a plurality of portable terminals; and
wherein the photographing order information, transmitted from one of the plurality of control apparatuses to one of the plurality of portable terminals, is prohibited from being transmitted from the control apparatus to another portable terminal.

According to the system or the method as described above, the photographing order information is displayed on a portable terminal. Therefore, even when medical photography is carried out at a doctor's round visit place such as a bed side or the like, the operator can easily check a patient to be photographed and a photographing condition by using a portable terminal. Accordingly, the photographing at the doctor' s round visit place can be efficiently performed. Furthermore, photographic order information transmitted from a control apparatus to a portable terminalisprohibitedfrom beingtransmitted to another portable terminal, so that the same photographic information can be prevented from being redundantly transmitted to plural portable terminals. Therefore, the redundant photographing can be prevented from being carried out on the same patient, and the photographing at the doctor' s round visit place can be accurately performed.

Preferably, in the above system, the photographing order information, transmitted from one of the plurality of control apparatuses to any one of the one or more portable terminals, is prohibited from being transmitted from another control apparatus to any one of the one or more portable terminals.

Preferably, the medical image photographing method further comprises:
prohibiting the photographing order information transmitted from one of the control apparatuses to any one of the one or more portable terminals, from being transmitted from another control apparatus to any one of the one or more portable terminals.

According to the system or the method described above, photographic order information, transmitted from a control apparatus to a certain portable terminal, is prohibited from being transmitted from another control apparatus so that the same photographic order information can be prevented from being redundantly transmitted to plural portable terminals. Accordingly, the redundant photographing based on the same photographic order information can be prevented, and the photographing at the doctor' s round visit place can be accurately performed.

Preferably, in the above system, when each of the plurality of control apparatuses is instructed so that the photographing order information transmitted to any one of the plurality of portable terminals is transmitted to any one of the plurality of portable terminals again, the control apparatus warns that the photographing order information has been already transmitted.

Preferably, in the above method, when each of the plurality of control apparatuses is instructed so that the photographing order information transmitted to any one of the plurality of portable terminals is transmitted to any one of the plurality of portable terminals again, the control apparatus warns that the photographing order information has been already transmitted.

According to the system or the method described above, it is warned that the photographic order information has been transmitted to some portable terminal. Therefore, the operator's attention can be attracted to the fact that the photographic order information has been already transmitted.

Preferably, in the above system, after the photographing order information is transmitted from one of the plurality of control apparatuses to any one of the plurality of portable terminals, another control apparatus deletes the photographing order information.

Preferably, in the above method, after the photographing order information is transmitted from one of the control apparatuses to any one of the plurality of portable terminals, another control apparatus deletes the photographing order information.

Preferably, the medical image photographing system further comprises a plurality of control apparatuses,
wherein each of the one or more portable terminals is connectable to a specific control apparatus of the plurality of control apparatuses.

Preferably, in the above system, the medical image generating apparatus uses an image recording panel whenever the patient is photographed;
the one or more portable terminals obtain recording panel identification information of each image recording panel, and store the photographing order information and the obtained recording panel identification information so as to set a correspondence of the photographing order information to the obtained recording panel identification information;
the one or more portable terminals transmits the stored photographing order information and the stored recording panel identification information to the specific control apparatuses;
the specific control apparatus transmits the photographing order information and the recording panel identification information received from the one or more portable terminals, to the information management apparatus; and
the information management apparatus stores the recording panel identification information so as to correspond to the photographing order information.

Preferably, the medical image photographing system further comprises an image information reading apparatus for reading out a photographing image from the image recording panel,
wherein the image information reading apparatus reads out the recording panel identification information of the image information recording panel, obtains relating information of the photographing order information, which includes a processing condition through a control apparatus, and reads out the image under the obtained processing condition.

Preferably, in the above system, when the photographing order information corresponding to the panel identification information is transmitted to the specific control apparatus, each of the one or more portable terminals transmits the photographing order information while adding information indicating that the photograph is finished, to the photographing order information.

Preferably, in the above system, each of the one or more portable terminals collates input patient identification information with patient identification information including the photographing order information, and notifies a collation result.

Preferably, in the above method, when the photographing order information corresponding to the panel identification information is transmitted to a specific control apparatus, each of the one or more portable terminals transmits the photographing order information while adding information indicating that the photograph is finished, to the photographing order information.

Preferably, the medical image photographing method further comprises:
inputting patient identification information to the portable terminal;
collating input patient identification information with patient identification information including the photographing order information; and
notifying a collation result.

According to the system or the method described above, even when the version of the specification of an apparatus used as a bed side in a hospital room is upgraded to expand the functions of the medical image photographing system, it is unnecessary to alter the overall medical image photographing system or the specifications of other apparatuses which are not used at the bed side in the hospital room. Furthermore, in this case, it is also unnecessary to change the specifications of other apparatuses which are used at the bed side, but has not been subjected to version upgrade. Therefore, the version upgrade to expand the functions of the medical image photographing system can be easily performed in low cost.

That is, the expansion of the functions of the medical image photographing system can be easily performed in low cost.

Preferably, the medical image photographing system further comprises a plurality of portable terminals,
wherein the photographing order information, transmitted from one of the plurality of control apparatuses to one of the plurality of portable terminals, is prohibited from being transmitted to another portable terminal.

According to the system described above, the photographic order information is displayed on the portable terminal. Therefore, even when photographing is carried out at a doctor's round visit place such as the bed side of a patient or the like, a photographing operator can easily check the patient to be photographed and the photographing condition by the portable terminal. Accordingly, the photographing at the doctor's round visit place can be efficiently performed. Furthermore, the photographic order information, transmitted from a control apparatus to a portable terminal, is prohibited from being transmitted to another portable terminal, and thus the same photographic order information can be prevented from being redundantly transmitted to plural portable terminals. Accordingly, the redundant photographing can be prevented from being carried out on the same patient, and the photographing at the doctor's round visit place can be accurately performed.

Preferably, in the above system, the photographing order information, transmitted from one of the plurality of control apparatuses to any one of the plurality of portable terminals, is prohibited from being transmitted from another control apparatus to any one of the plurality of portable terminals.

According to the system described above, the photographic order information transmitted from a control apparatus to some portable terminal is prohibited from being transmitted from another control apparatus. Therefore, the same photographic order information can be prevented from being redundantly transmitted to plural portable terminals. Accordingly, the redundant photographing based on the same photographic order information can be prevented, and the photographing at the doctor's round visit place can be accurately performed.

Preferably, in the above system, when each of the plurality of control apparatuses is instructed so that the photographing order information transmitted to any one of the plurality of portable terminals is transmitted to any one of the plurality of portable terminals again, the control apparatus warns that the photographing order information has been already transmitted.

According to the system described above, it is warned that the photographic order information has been transmitted to some portable terminal. Therefore, the operator's attention can be attracted to the fact that the photographic order information has been already transmitted.

Preferably, in the above system, after the photographing order information is transmitted from one of the plurality of control apparatuses to any one of the plurality of portable terminals, another control apparatus deletes the photographing order information.

### BRIEF DESCRIPTION OF THE DRAWINGS

The present invention will become more fully understood from the detailed description given hereinbelow and the accompanying drawing which are given by way of illustration only, and thus are not intended as a definition of the limits of the present invention, and wherein;
FIG. 1 is a block diagram showing a medical image photographing system 100 according to a first embodiment to which the present invention is applied;
FIG. 2 is a block diagram showing the internal construction of an information management apparatus 110 in a radiographing room shown in FIG. 1;
FIG. 3 is a block diagram showing the internal construction of a portable terminal 140 shown in FIG. 1;
FIG. 4 is a block diagram showing the internal construction of control apparatuses 160a, 160b shown in FIG. 1;
FIG. 5 is a flowchart showing information processing of the information management apparatus 110 in the radiographing room shown in FIG. 1;
FIG. 6 is a flowchart showing the information processing of a portable terminal 140 shown in FIG. 1;
FIG. 7 is a block diagram showing the information processing of control apparatuses 160a, 160b shown in FIG. 1;
FIG. 8 is a diagram showing the system construction of a medical image photographing system according to a second embodiment to which the present invention is applied;
FIG. 9 is a diagram showing the functional construction of a control apparatus 240;
FIG. 10 is a diagram showing the data structure of a radiographing order information file 2461;
FIG. 11 is a diagram showing the data structure of a registered information file 2462;
FIG. 12 is a diagram showing the functional construction of a portable terminal 260;
FIG. 13 is a flowchart showing radiography preparation processing executed by a control apparatus 240;
FIG. 14A shows a menu screen 2431a when normal radiography is selected, and FIG. 14B shows a menu screen 2431b when portable radiography is selected;
FIG. 15 shows a patient list screen 2432;
FIG. 16 is a flowchart showing new registration processing executed by the control apparatus 240;
FIG. 17 shows an input screen 2433 for patient information;
FIG. 18 shows a selection screen 2434 for radiographing condition;
FIG. 19 is a flowchart showing order transmission processing executed by the control apparatus 240;
FIG. 20A shows a check screen 2435 for checking a transmission/reception status of radiographing order information, FIG. 20B is a diagram showing a mark displayed in the transmission/reception status b5 when the radiographing order information has been transmitted, and FIG. 20C is a diagram showing a mark displayed in the transmission/reception status b5 when cassette registration is received for the radiographing order information transmitted;
FIG. 21 is a flowchart showing the cassette registration processing executed by the portable terminal 260;
FIG. 22A shows a patient list screen 2631, and FIG. 22B shows an order list screen 2632;
FIG. 23 is a flowchart showing the image registration processing executed by the control apparatus 260;
FIG. 24 shows an image check screen 2437;
FIG. 25 is a block diagram showing the construction of a medical image system 300 according to a third embodiment to which the present invention is applied;
FIG. 26 is a block diagram showing the internal construction of an information management apparatus 310 shown in Fig. 25;
FIG. 27 is a block diagram showing the internal construction of a control apparatus 360 shown in FIG. 25;
FIG. 28 is a flowchart showing information processing executed by the information management apparatus 310 shown in FIG. 25;
FIG. 29 is a flowchart showing communication processing executed by a portable terminal 340 shown in FIG. 25;
FIG. 30 is a flowchart showing the communication processing executed by the control apparatus 360 shown in FIG. 25;
FIG. 31 is a flowchart showing the image processing executed by the control apparatus 360 shown in FIG. 25;
FIG. 32A shows a display of patient's name/ID in the radiographing order information on the screen of the portable terminal 340 shown in FIG. 25, and FIG. 32B shows a display of radiographing condition/panelidentificationinformation every patient in the radiographing order information on the screen of the portable terminal shown in FIG. 25;
FIG. 33A shows a display of a list of radiographing order information on the screen of the control apparatus 360 shown in FIG. 25, FIG. 33B is a diagram showing a mark displayed when radiographing order information is transmitted from the control apparatus 360 to the portable terminal 340, and FIG. 33C is a diagram showing a mark displayed when radiographing order information on which radiography has been carried out is transmitted from the portable terminal 340;
FIG. 34 shows an example of a radiograph displayed on the screen of the control apparatus 360 shown in FIG. 25;
FIG. 35 is a diagram showing the system construction of a medical image radiographing system 400 according to a fourth embodiment to which the present invention is applied;
FIG. 36 is a diagram showing the functional construction of a control apparatus 440;
FIG. 37 is a flowchart showing order transmission processing executed by the control apparatus 440; and
FIG. 38 is a diagram showing a conventional medical image system.

### DESCRIPTION OF THE PREFERRED EMBODIMENTS

### [First Embodiment]

A first embodiment to the present invention is applied will be described in detail with reference to the drawings.

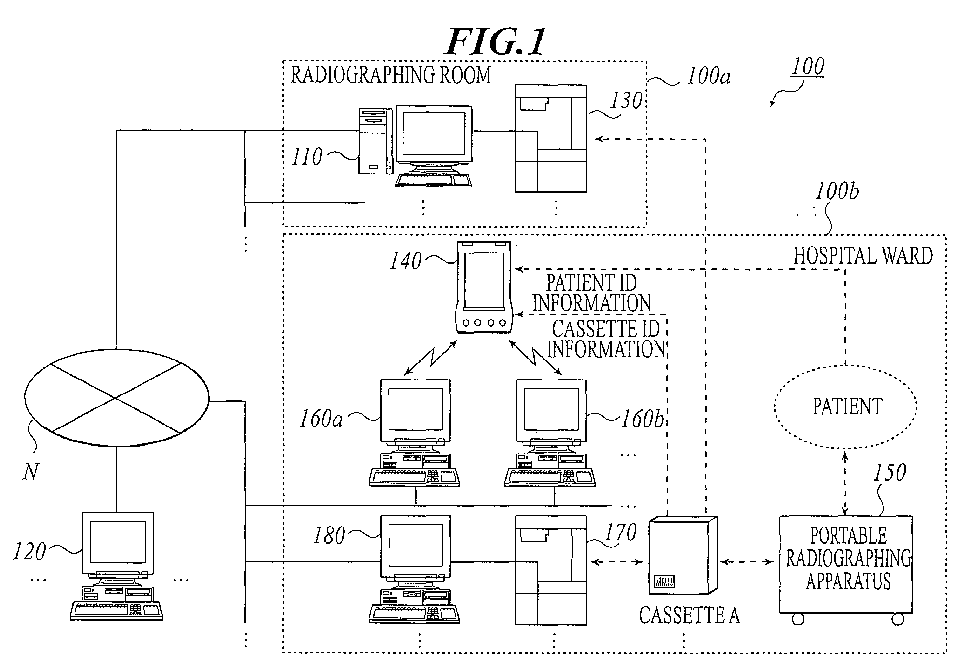

First, the construction of a medical image radiographing system 100 will be described with reference to FIG. 1.

FIG. 1 is a block diagram showing the construction of the medical image photographing system 100. As shown in FIG. 1, the medical image photographing system 100 is equipped with an information management apparatus 110 and an image information reading apparatus 130 in a radiographing room 100a, and also with a portable radiographing apparatus 150, control apparatuses 160a, 160b, an image information reading apparatus 170 and an image processing device 180 in a hospital ward 100b.

The information management apparatus 110, the control apparatuses 160a, 160b, the image processing device 180, etc. are connected to a reserving apparatus 120 through a network N such as LAN (Local Area Network), WAN (Wide Area Network) or the like which is constructed in a hospital (containing related facilities), and they can transmit/receive data mutually. The information management apparatus 110 is connected to the image information reading apparatus 130 in the radiographing room 100a, and the image processing device 180 is connected to the image information reading apparatus 170 in the hospital ward 100b. The portable terminal 140 is connectable to the control apparatuses 160a, 160b through an interface such as RS-232C, IrDA (Infrared Data Association) or wireless LAN to transmit/receive data mutually. The portable terminal 140 is also connectable to the network N. The portable terminal is PDA or the like.

A plurality of information management apparatuses 110 described above and a plurality of image information reading apparatus 130 described above may be set up in the radiographing room 100a, and also a plurality of image processing devices 180 and a plurality of image information reading apparatus 170 may be set up in the hospital ward (for example, they can be set up at each dedicated space or each nurse station equipped on each floor (not shown)). Apluralityof control apparatuses 160a, 160b can be set in the hospital ward 100b, and it is preferable that at least one control apparatus 160a (160b) is set up every hospital room. The reserving apparatus 120 is equipped to a reception room for diagnosis or each medical examination room, and the portable terminal 140 is carried by a radiographing operator, and plural portable terminals 140 are available.

The information management apparatus 110 controls the image information reading apparatus 130 to read out radiographs from a radiographed cassette such as a cassette A or the like. Upon receiving radiographing order information input through the reserving apparatus 120, the information management apparatus 110 records the radiographing order information into a radiographing order information file 1171. The information management apparatus 110 stores into an image DB 1172 the radiograph data read by the image information reading apparatus 130, 170.

The construction of the image management apparatus 110 will be described in detail later.

The reserving apparatus 120 is an input terminal for inputting radiographing order information for radiography by an examining doctor or the like, and a terminal PC equipped to each medical examination room or a diagnosis reception room serves as the reserving apparatus 120. The reserving apparatus 120 transmits the input radiographing order information to the information management apparatus 110.

The image information reading apparatus 130 reads out the radiographs from the radiographed cassette such as the cassette A or the like under the control of the information management apparatus 110. Here, the cassette A is equipped with an imaging plate having a photostimulable phosphor layer formed thereon (not shown). That is, the image information reading apparatus 130 induces emission of fluorescence corresponding to accumulated radiation energy from the photostimulable phosphor of the imaging plate, photoelectrically converts the fluorescence thus emitted, and outputs radiograph data thus achieved.

Here, a radiographing panel on which a radiograph achieved through medical radiography is recorded is implemented by the cassette A or the imaging plate equipped to the cassette A.

Furthermore, a bar code inherent to the cassette A is attached as ID information (hereinafter referred to as cassette ID information) on the surface of the cassette A. The cassette ID information of the cassette A is not limited to the bar code, and any identification material may be used insofar as the cassette A can be identified by the material. For example, an IC chip in which cassette ID information is recorded may be used. Furthermore, in place of the cassette ID information, a bar code as ID information of the imaging plate equipped to the cassette may be attached to the surface of the imaging plate.

Here, the inherent panel identification information is implementedby the cassette ID information. The portable terminal 140 achieves the cassette ID information and the patient ID information, and also achieves the radiographing order information connected with the patient indicated by the patient ID information from the control apparatus 160a, 160b, etc.. or the image processing device 180. Here, the patient ID information is given by a bar code printed on a patient name plate, a patient diagnosis card, a patient carte or the like. The patient ID information contained in the radiographing order information corresponds to the information represented by the bar code.

The portable terminal 140 adds the radiographing order information thus achieved with the cassette ID information, and then transmit the information thus achieved to the control apparatus 160a, 160b, etc.. or the image processing device 180. In this case, the control apparatus 160a, 160b, etc.. or the image processing device 180 which relays when the portable terminal 140 achieves the radiographing order information from the information management apparatus 110 is not required to be identical to the control apparatus 160a, 160b, etc.. or the image processing device 180 which relays when the portable terminal 140 transmits the radiographing order information added with the cassette ID information to the information management apparatus 110. That is, when achieving the radiographing order information from the control apparatus 160a, the portable terminal 140 can add the radiographing order information with the cassette ID information, and transmit the radiographing order information thus achieved to the control apparatus 160b different from the control apparatus 160a or the image processing device 180.

The portable terminal 140 may be a portable type communication terminal, and it may be a laptop type PC or the like.

The construction of the portable terminal 140 will be described in detail layer.
The portable radiographing apparatus 150 is a radiographing apparatus movable between hospital rooms or between beds, and radiography is carried out by using a cassette A. The control apparatus 160a, 160b, etc.. is set up on each floor or in each hospital room in the hospital ward 100b, and it transmits/receives data to/from plural portable terminals containing the portable terminal 40, etc.

The construction of the control apparatus 160a, 160b, etc.. will be described in detail later.

The image information reading apparatus 170 has the same construction as the image information reading apparatus 130, and set up on each floor or at each nurse station in the hospital ward 100b. The image processing device 180 is set up together with the image information reading apparatus 170, and controls the image processing device of the image information reading apparatus 170. The image processing device 180 can display particularly radiographing image data, so that the radiography operator can easily check the radiographing result without moving on the floor or between floors.

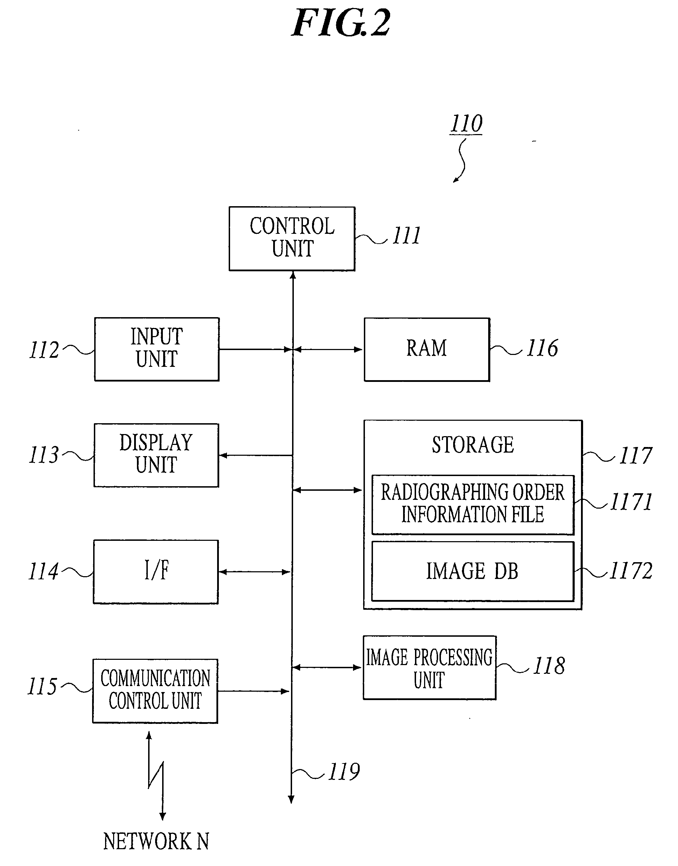

Next, the construction of each of the information management apparatus 110, the portable terminal 140 and the control apparatus 160a will be described in detail with reference to FIGS. 2 to 4.

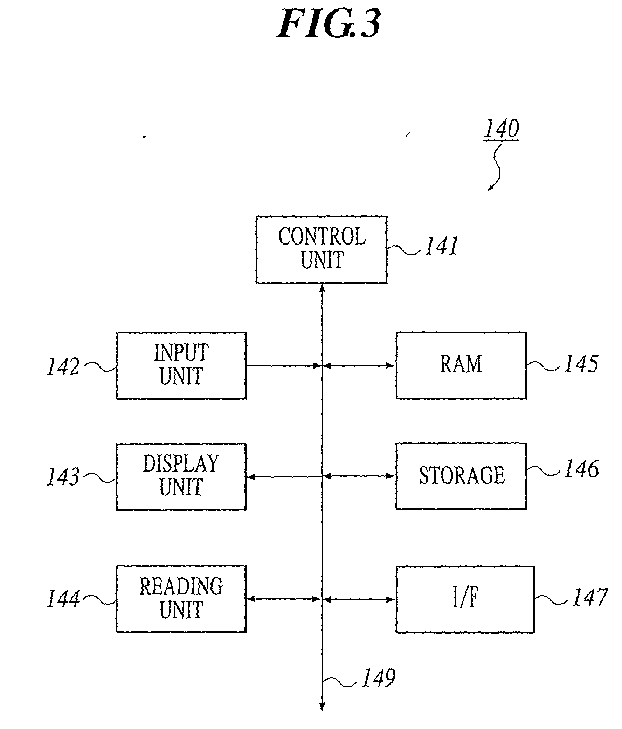

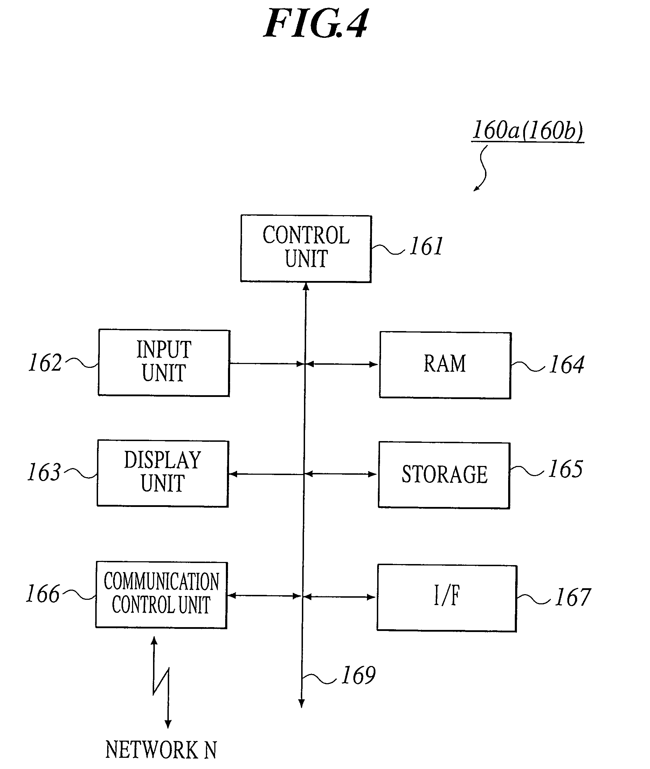

FIG. 2 is a block diagram showing the internal construction of the information management apparatus 110, FIG. 3 is a block diagram showing the internal construction of the portable terminal 140, and FIG. 4 is a block diagram showing the internal construction of the control apparatus 160a. Here, the control apparatus 160b has the same construction as the control apparatus 160a, and thus the illustration and description of the construction of the control apparatus 160b are omitted to simplify the illustration and the description.

First, the construction of the information management apparatus 110 will be described.

As shown in FIG. 2, the information management apparatus 110 is equipped with a control unit 111, an input unit, a display unit 113, I/F 114, a communication control unit 115, RAM 116, a storage 117, an image processing unit 118, etc., and these parts are connected to one another through a bus 119.

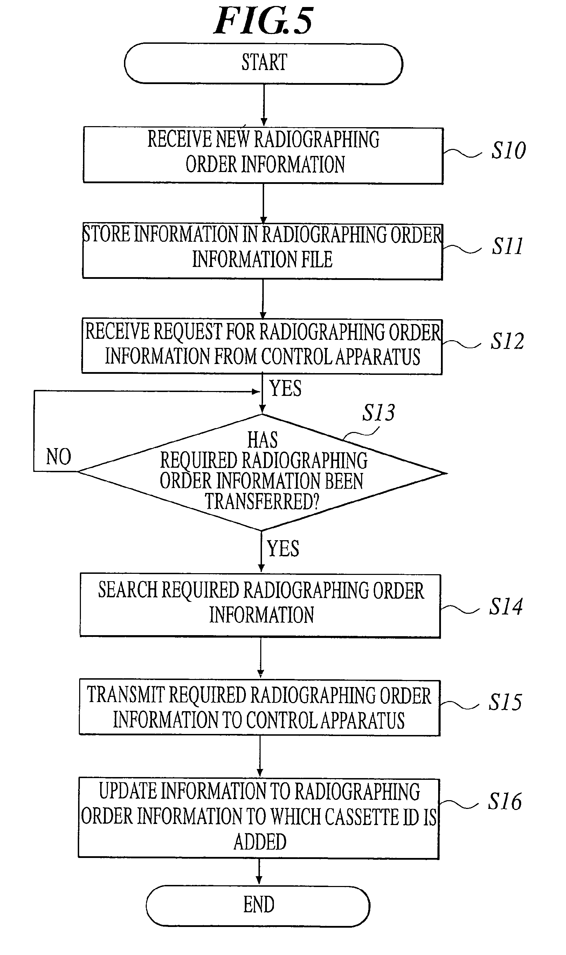

The control unit 111 has CPU (Central Processing Unit) (not shown) , and it reads out and executes various programs stored in the storage 117. Particularly, the control unit 111 reads out from the storage 117 the program for executing the information processing represented by the flowchart of FIG. 5 described in detail layer and executes the program thus read out.

The control unit 111 controls the image reading processing of the image information reading apparatus 130 on the basis of the radiographing order information. Furthermore, the control unit 111 selects a processing pattern (containing frequency processing, gradation processing, rotation processing, enlarging/reducing processing, etc.) corresponding to a radiographing condition described in the radiographing order information connected with the radiographing image data which are read out from the image information reading apparatus 130 or from the image information reading apparatus 170 set up in the hospital ward 100b, and makes the image processing unit 118 execute the image processing based on the processing pattern concerned. The radiographing images on which the image processing has been carried out by the image processing unit 118 are stored in the image DB 1172 in association with the patient ID information or radiographing order information by the control unit 111. Here, the processing pattern information corresponding to the radiographing condition is stored in the storage 117 in advance.

The control unit 111 renews the radiographing order information of the radiographing order information file 1171 as occasion demands. For example, upon receiving the radiographing order information added with the cassette ID information from the control apparatus 160a, 160b, etc.., the control unit 111 renews the corresponding radiographing order information recorded in the radiographing orderinformationfile 1171 to the radiographing order information received.

The input unit 112 is equippedwith a keyboardhaving cursor keys, numeric input keys, various function keys, etc., and a pointing device such as a mouse or the like (all are not shown) , and outputs to the control unit 111 an instruction signal input through the key operation of the key board or the mouse operation. The input unit 112 may have a touch panel equipped to the screen of the display unit 113, and outputs an instruction signal input through the touch panel to the control unit 111.

The display unit 113 has LCD (Liquid Crystal Display), CRT (Cathode Ray Tube) or the like (not shown), and displays each kind of display data in accordance with a display control signal from the control unit 111.

I/F 114 is an interface for connecting the information management apparatus 110 and the image information reading apparatus 130 so that data can be communicated therebetween.

The communication control unit 115 is equipped with an LAN board, a router or TA (Terminal Adapter) , etc. (all elements are not shown), and controls the communications carried out between respective apparatuses connected to each other through a network N.

RAM (Random Access Memory) 116 develops various kinds of programs read out from the storage 117 by the control unit 111 so that the programs can be executed, and then stores these programs therein. RAM 116 temporarily stores various kinds of data generated when the programs are executed by the control unit 111.

The storage 117 is equipped with a freely-writable non-volatile semiconductor memory such as HDD (Hard Disc Drive), and stores various programs and various data therein. The storage 117 particularly stores a program for executing the information processing represented by the flowchart of FIG. 5 described in detail later. Further, the storage 117 stores a plurality of processing pattern for executing the image processing in accordance with a radiographing condition.

The storage 117 has the radiographing order information file 1171 and the image DB 1172. The radiographing order information file 1171 records radiographing order information input through the reserving apparatus 120, and in association with the patient ID information or the radiographing order information, the image DB 1172 stores the radiographing image data which have been subjected to the image processing in the information management apparatus 110 (or image processing device 180).

The storage 117 may be designed to have a recording medium (not shown) in which various kinds of programs and various kinds of data are stored in advance. The recording medium is fixedly or freely detachably equipped to the storage 117, and it is a non-volatile memory formed of a magnetic recording medium, an optical recording medium or a semiconductor memory.

Under the control of the control unit 111, the image processing unit 118 selects the processing pattern corresponding to a radiographing condition described in the radiographing order information connected to the radiographing image data which are read out from the image information reading apparatus 130 or from the image information reading apparatus 170 set up in the hospital ward 100b, and carries out the image processing (containing compression/expansion processing) based on the processing pattern.

Next, the construction of the portable terminal 140 will be described.

As shown in FIG. 3, the portable terminal 140 is equipped with a control unit 141, an input unit 142, a display unit 143, a reading unit 144, RAM 145, a storage 146, I/F 147, etc., and these elements are connected to one another through a bus 149.

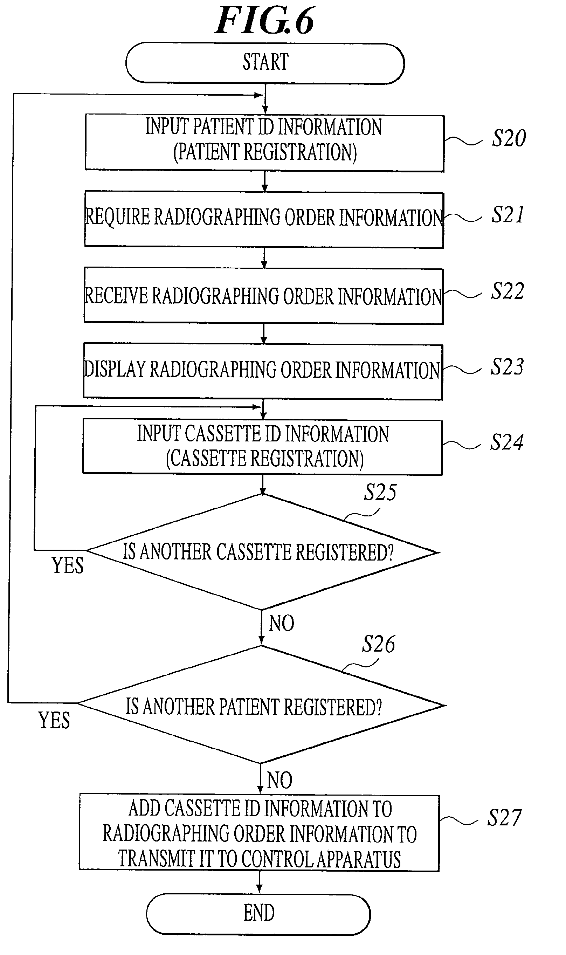

The control unit 141 has CPU (not shown), and reads out and executes various kinds of programs stored in the storage 146. Particularly, the control unit 141 reads out from the storage unit 146 a program for executing the information processing represented by the flowchart of FIG. 6 described in detail layer and executes the program thus read out.

The input unit 142 has cursor keys, numeric input keys, various function keys, etc. (all the elements are not shown), and outputs an instruction signal input through the key operation of each key to the control unit 141. The input unit 142 may be equipped with a touch panel equipped to the screen of the display unit 143, and outputs an instruction signal input through the touch panel to the control unit 141.

The display unit 143 has LCD (not shown) or the like, and displays various kinds of display data in accordance with a display control signal from the control unit 141. Particularly, the display unit 143 displays radiographing order information achieved from the control apparatus 160a, 160b, etc.. or the image processing device 180.

The reading unit 144 has a bar code reader (not shown), and outputs bar code information read out by the bar code reader to the control unit 141.

RAM 145 develops various kinds of programs read out from the storage unit 146 so that the programs can be executed, and then stores the programs therein. RAM 145 temporarily stores various kinds of data generated when the programs are executed by the control unit 141 (for example, the patient ID information, the cassette ID information and the radiographing order information read through the reading unit 144).

The storage 146 is equipped with a non-volatile semiconductor memory such as HDD or the like, and stores various kinds of programs and various kinds of data. Particularly, the storage unit 146 stores therein a program for executing the information processing represented by the flowchart of FIG. 6 described in detail later. Furthermore, the storage 146 stores plural processing patterns for executing the image processing in conformity with the radiographing condition.

The storage 14 6 may be designed to have a recording medium in which various kinds of programs and various kinds of data are stored in advance. The recording medium is a non-volatile memory which is fixedly or freely detachably equipped to the storage 146 and is equipped with a magnetic recording medium, an optical recording medium or a semiconductor memory.

I/F 147 is an interface adapted to RS-232C, IrDA or wireless LAN to connect the portable terminal 140 and the control apparatus 160a, 160b, etc.

Next, the construction of the control apparatus 160a will be described.

As shown in FIG. 4, the control apparatus 160a is equipped with control unit 161, an input unit 162, a display unit 163, a communication control unit 164, RAM 165, a storage 166 and I/F 167, and these elements are connected to one another through a bus 169.

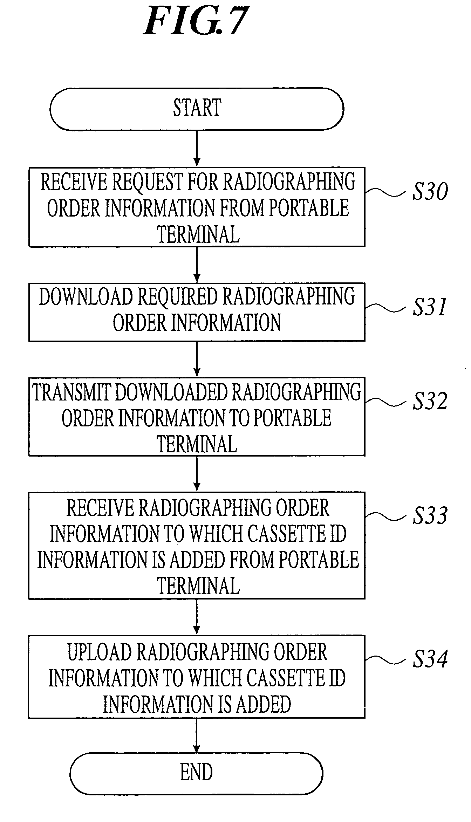

The control unit 161 has CPU (not shown), and reads out and executes various kinds of programs stored in the storage 165. Particularly, the control unit 161 reads out from the storage 165 a program for executing the information processing represented by the flowchart of FIG. 7 described in detail later, and then executes the program thus read out.

The input unit 162 is equipped with a keyboard having cursor keys, numeric input keys, various function keys, etc. and a pointing device such as a mouse or the like (all the elements are not shown), and outputs an instruction signal input through the key operation of the keyboard or the mouse operation to the control unit 161. The input unit 162 may have a touch panel equipped to the screen of the display unit 163, and outputs an instruction signal input through the touch panel to the control unit 161.

The display unit 163 has LCDorCRT (not shown), and displays various kinds of display data in accordance with a display control signal from the control unit 161.

RAM 164 develops the various kinds of programs read out from the storage 165 by the control unit 161 while the programs can be executed, and then stores these programs therein. RAM 164 temporarily stores various kinds of data generated when the programs are executed by the control unit 161.

The storage 165 is equipped with a non-volatile semiconductor memory such as HDD or the like, and stores various kinds of programs and various kinds of data therein. Particularly, the storage unit 165 stores a program for executing the information processing represented by the flowchart of FIG. 7 described in detail later.

The storage 165 may be designed to have a recording medium (not shown) in which various kinds of programs and various kinds of data are stored in advance. The recording medium is a non-volatile memory which is fixedly or freely detachably equipped to the storage 165 and is equipped with a magnetic recording medium, an optical recording medium or a semiconductor memory.

The communication control unit 166 is equipped with a LAN board, a router or TA (Terminal Adapter) (not shown) , and controls the communications among the respective apparatuses connected to one another through the network N.

I/F 167 is an interface adapted to RS-232C, IrDA or wireless LAN for connecting the control apparatus 160a and the portable terminal 140.

Next, the operation of each of the information management apparatus 110, the portable terminal 140 and the control apparatus 160a will be described with reference to FIGs. 5 to 7.

FIG. 5 is a flowchart showing the information processing executed in the information management apparatus 110, FIG. 6 is a flowchart showing the information processing executed by the portable terminal 140, and FIG. 7 is a flowchart showing the information processing executed by the control apparatus 160a. The operation of the control apparatus 160b is the same as the control apparatus 160a, and description of the operation of the control apparatus 160b is omitted from the following description in order to simplify the description.

First, the operation of the information management apparatus 110 will be described.

Upon receiving new radiographing order information through the reserving apparatus 120 (step S10) , the control unit 111 records the new radiographing order information concerned into the radiographing order information file 1171 (step S11).

Upon receiving a transmission demand (request) for radiographing order information, the control unit 111 and ID information of the portable terminal 140 serving as the requester concerned from the control apparatus 160a, 160b, etc.. (step S12), the control unit 111 judges whether the radiographing order information has been already transmitted (step S13). If the radiographing order information has been transmitted (step S13:Yes), the processing is finished. If it has not yet been transmitted (step S13: No), the radiographing order information thus requested is searched from the radiographing order information file 1171 with the patient ID information as a key for the search (step S14).

The control unit 111 transmits the radiographing order information searched in step S14 to the control apparatus 160a, 160b, etc.. of the requester (that is, the portable terminal 140 serving as the requester) (step S15). At this time, the control unit 111 adds the radiographing order information concerned with the ID information of the portable terminal 140 serving as a transmission destination of the radiographing order information thus transmitted, and records this composite information into the radiographing order information file 1171. The control unit 111 judges in step S13 whether the ID information of the portable terminal 140 serving as the transmission destination is added to the radiographing order information of the radiographing order information, and on the basis of this judgment, the control unit 111 judges whether the radiographing order information has been already transmitted or not.

Upon receiving the radiographing order information added with cassette ID information from the control apparatus 160a, 160b, etc.., the control unit 111 renews the corresponding radiographing order information in the radiographing order information file 1171 to the radiographing order information added with the cassette ID information thus received (step S16) .

The processing of the step S14 may be omitted, and in connection with this omission, the ID information of the portable terminal serving as the transmission destination of the radiographing order information is not necessarily recorded in the radiographing order information file 1171.

Next, the operation of the portable terminal 140 will be described.

Upon achieving through the reading unit 144 the patient ID information of a patient to be radiographed (step S20), the control unit 141 transmits the patient ID information concerned to the control apparatus being currently connected to the control unit 141 (for example, the control apparatus 160a), and requests the radiographing order information for the patient (step S21).

Upon receiving the radiographing order information requested in step S21 from the control apparatus 160a (step S22), the control unit 141 displays the radiographing order information thus received on the display unit 143 (step S23).

The control unit 141 reads out the cassette ID information (bar code) through the reading unit 144 in connection with the radiographing condition described in the radiographing order information received in step S22, and stores the read-out cassette ID information in association with the radiographing condition concerned into RAM 145 (step S24).

When there is another radiographing condition described in the radiographing order information concerned (step S25 : Yes), the control unit 141 returns to step S24 to read through the reading unit 144 the cassette ID information (bar code) of another cassette different from the cassette A in connection with the other radiographing condition, and stores the read-out cassette ID information in association with the radiographing condition concerned into RAM 145. The control unit 141 repeats the processing of the step S24 at the frequency corresponding to the number of the radiographing conditions described in the radiographing order information.

When registration of the cassette is finished (step S25; No), if there are other patients to be radiographed (step S26; Yes), the control unit 141 goes to step S20 to repeat each processing of the steps S20 to S25 on the patients at the frequency corresponding to the number of patients to be radiographed.

When the registration of the cassette and the patients is finished (step S26; No), the control unit 141 combines the radiographing order information and the cassette ID information registered every radiographing condition, and transmits the combined information as new radiographing order information to the control apparatus (for example, the control apparatus 160b).

Here, it is assumed that the portable terminal 140 is connected to the control apparatus 160a at the stage of the step S21 and it is newly connected to the control apparatus 160b at the stage of the step S27. The portable terminal 140 can be connected to each of the control apparatus 160a and the control apparatus 160b so that the data communications can be made therebetween, and also can be connected to control apparatuses (not shown) other than the control apparatuses 160a, 160b and the image processing device 180, so that the processing of the steps S21, S22, S27 can be executed.

Next, the operation of the control apparatus 160a will be described. It is assumed that the portable terminal 140 is connected to the control apparatus 160a.

When radiographing order information is requested from the portable terminal 140 (step S30), the control unit 161 downloads the requested radiographing order information from the information management apparatus 110 (step S31).

The control unit 161 transmits the radiographing order information downloaded in the step S31 to the portable terminal 140 as the requester (step S32).

Furthermore, upon receiving radiographing order information added with cassette ID information from the portable terminal 140 (step S33), the control unit 161 uploads the radiographing order information to the information management apparatus 110 (step S34).

As described above, the medical image photographing system 100 which can carry out radiography in a hospital room is equipped with the control apparatuses 160a, 160b, etc. which are set up in hospital rooms of a hospital ward or at nurse stations and mediate between the portable terminal 140 for managing radiographing order information and the cassette ID information of a cassette adapted to each radiographing condition and the information management apparatus 110 in which the radiographing order information is stored. When the radiographing order information is requested from the portable terminal 140, the control apparatus 160a, 160b, etc. downloads the radiographing order information from the informationmanagement apparatus 110, and transmits the information to the portable terminal 140. When radiographing order information added with cassette ID information is transmitted from the portable terminal 140, the control apparatus 160a, 160b, etc.. uploads the radiographing order information added with the cassette ID information to the information management apparatus 110. The control apparatuses 160a, 160b, etc. have the same construction, and the portable terminal 140 could perform the same data transmission/reception even if it is connected to any control apparatus.

Therefore, when radiography is carried out in a hospital room, the radiographing condition and the cassette can be easily associated with each other by using the portable terminal 140 without using any order sheet (paper). Once the radiographing condition and the cassette are associated with each other, the cassette ID information is added to the radiographing order information, and stored in the radiographing order file 1171 of the information management apparatus 110, whereby the image reading processing of reading data from the cassette and the image processing for the read-out data can be automatically properly carried out on the basis of the radiographing order information without operator' s work of selecting a cassette from which images should be read out or manually inputting data while checking an order sheet. In addition, an unanticipated situation which may be induced by the operator or the like can be avoided, and safety can be enhanced.

Furthermore, the control apparatus 160a, 160b connected to the network N is set up in each hospital room or at each nurse station, and the portable terminal 140 can connect to any of the control apparatuses 160a, 160b so that the data communication can be made. Therefore, the radiographing operator can easily achieve radiographing order information through a near control apparatus without going around for a connectable control apparatus, and also the radiographing operator can easily return radiographing order information added to cassette ID information to the information management apparatus 110. Accordingly, information for identifying which cassette is used in radiography for which patient under which radiographing condition (i.e., information for identifying a cassette, a patient and a radiographing condition under radiography) can be commonly owned in the overall hospital on a real-time basis. Furthermore, the radiographing operator can check the radiographing result through the image processing device 180 by merely loading the cassette in the image information reading apparatus 170 and starting the image reading processing. Therefore, the operator can immediately judge whether radiography should be carried out again.

Furthermore, since the ID information of the portable terminal 140 achieving the radiographing order information is managed by the information management apparatus 110, there can be avoided such a situation that the same radiographing order information is transmitted to a different portable terminal.

That is, according to the medical image photographing system 100 to which the present invention is applied, the radiographing operation in a hospital room and the image processing on radiographs achieved through the radiography can be easily and efficiently carried out.

The description of the above embodiment is related to an example of the medical image photographing system according to the present invention, and the present invention is not limited to the above embodiment. The detailed construction and the detailed operation of the medical image photographing system 100 according to this embodiment may be appropriately modified without departing from the subject matter of the present invention.

According to the present invention, it is unnecessary to fill the order content for plural items in plural order sheets every patient and radiographing condition, and thus there can be avoided such an unanticipated situation that the processing associated with the X-ray photographing is made cumbersome or erroneous data are filled in order sheets. Furthermore, there can be avoided such a situation that the order sheets are torn or lost while the radiographing operator frequently moves in or out of the radiographing room or comes and goes among plural hospital rooms.

Furthermore, in such a case that at least one control apparatus is set up on each floor in a hospital ward, a radiographing operator is not needed to move on a floor or between floors to achieve radiographing order information and look for a control apparatus, and the operator can easily achieve the radiographing order information from any control apparatus set up on the same floor as the hospital room of a patient to be radiographed. In this case, radiographing order information containing panel identification information inherent to a radiographingpanel fromany control apparatus to the information management apparatus.

That is, the radiographing operation in the hospital room and the image processing on radiographs achieved through the radiographing operation can be easilyand efficiently performed.

### [Second Embodiment]

In the first embodiment, the description has been made to the medical image photographing system in which portable terminals and control apparatuses are connected to one another in a connection mode of M:N and the association (correspondence relationship) between a radiographing condition and a cassette can be easily established by using a portable terminal in such a connection mode that the portable terminal is directly connected to a network and can transmit/receive data to/from any control apparatus. In the second embodiment, the description will be made to a medical image photographing system in which radiographing order information transmitted from a control apparatus to a portable terminal is prohibited from being transmitted to another portable terminal in the case where portable terminals and control apparatuses are connected to one another in the connection mode of M:N.

The details of the second embodiment of the present invention will be described hereunder in detail with reference to the accompanying drawings.

In this embodiment, plural portable terminals for displaying radiographing order information at a doctor's round visit place are equipped to the medical image photographing system, radiographing order information is transmitted from a control apparatus to a certain portable terminal to display the radiographing order information on the portable terminal concerned, and a flag indicating that radiographing order information transmitted from a control apparatus to a certain portable terminal has been already transmitted is set to thereby prohibit transmission of the flag-set radiographing order information to another portable terminal.

First, the construction of the second embodiment will be described.

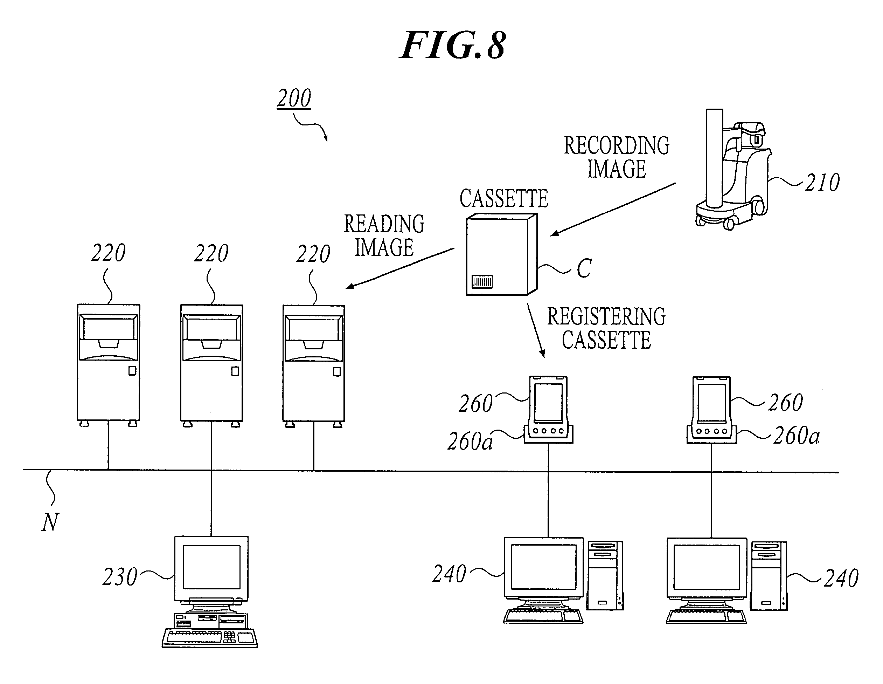

FIG. 8 shows the system construction of a medical image photographing system 200 according to this embodiment.

As shown in FIG. 8, the medical image photographing system 200 of this embodiment includes a portable radiographing apparatus 210, cassette-dedicated reading apparatus 220, an information management apparatus 230, control apparatuses 240 and portable terminals 260, etc, and the reading apparatus 220, the information management apparatus 230 and the control apparatuses 240 are connected to one another through a communication network N. The portable terminal 260 is connected to the communication network through the communication terminal 260a, and connected to any control apparatus 240 so that data communications with any control apparatus 240 can be performed. The setup numbers of the control apparatuses 240 and the portable terminals 260 may be one or plural, and it is not limited to a specific value.

The portable radiographing apparatus 210 is a movable radiographing apparatus having a radiation source, and it radiographs a patient at a doctor's round visit place (hereinafter referred to as "portable radiograph") to achieve a medical image (radiograph) and records the medical image on a cassette c. The cassette c contains a phosphor plate and records on the phosphor plate the medical image achieved through radiography by the portable radiographing apparatus 210. Identification information (hereinafter referred to as "cassette ID") for identifying (discriminating) the cassette c concerned from other cassettes c is provided on the surface of the cassette c in the form of a bar code.

The reading apparatus 220 reads out the medical image recorded in the cassette c according to an instruction of the control apparatus 240. The medical image read out from the cassette c and the cassette ID of the cassette c are transmitted to the control apparatus 240.

The information management apparatus 230 accepts a request from a doctor, issues radiographing order information, and adds the radiographing order information thus issued with identification information (hereinafter referred to as "order ID") for identifying the radiographing order information from other radiographing order information, thereby managing the radiographing order information. The information management apparatus 230 may be an accept terminal for accepting issuance of radiographing order information or an information management system such as HIS, RIS or the like.

The information management apparatus 230 transmits the issued radiographing order information to each control apparatus 240 simultaneously, and also stores the medical image transmitted from each control apparatus 240 and the radiographing order information in association with each other, whereby the images are managed.

The information management apparatus 230 manages the transmission status of radiographing order information to the portable terminal 260 in each control apparatus 240. That is, the information management apparatus 230 receives from each control apparatus 240 the order ID of the transmitted radiographing order information from the control apparatus 240 to the portable terminal 260, andmanages the transmission status as to whether the radiographing order information has been already transmitted. When receiving an inquiry about the transmission status of the radiographing order information from each control apparatus 240, the information management apparatus 230 transmits to the control apparatus 240 the order ID of the radiographing order information which has been already transmitted.

The control apparatus 240 controls the reading operation of the medical image in the reading apparatus 220, and the medical image thus read out is received from the reading apparatus 220, and the radiographing order information and the medical image are associated with each other (hereinafter referred to as "image registration").

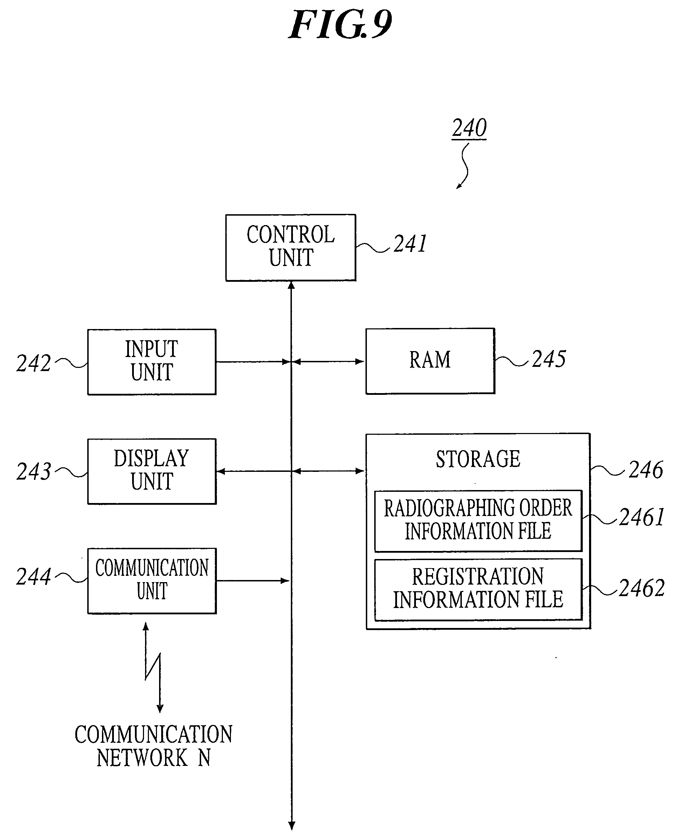

FIG. 9 shows the functional construction of the control apparatus 240.

As shown in FIG. 9, the control apparatus 240 is equipped with a control unit 241, an input unit 242, a display unit 243, a communication unit 244, RAM (Random Access Memory) 245 and a storage 246.

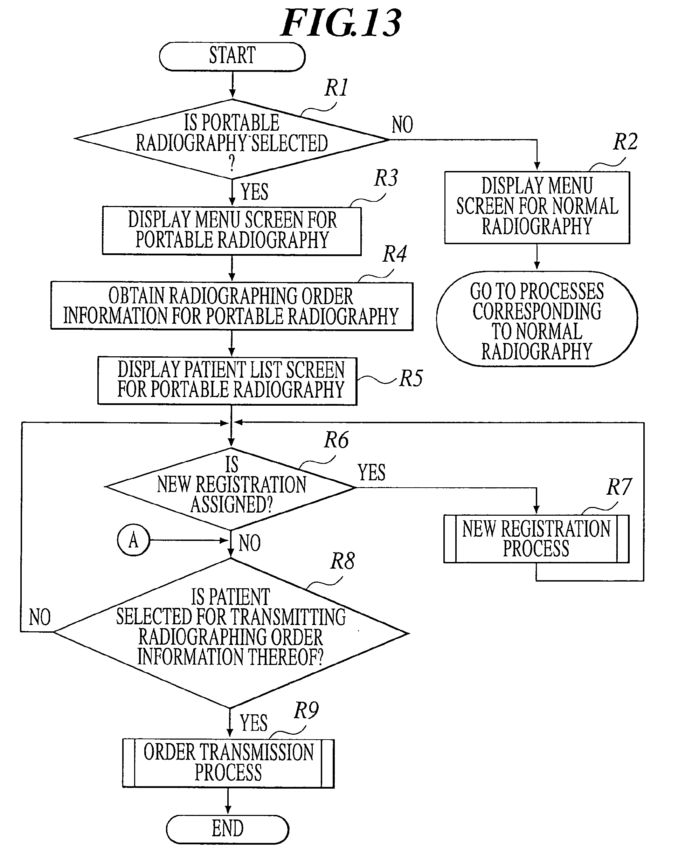

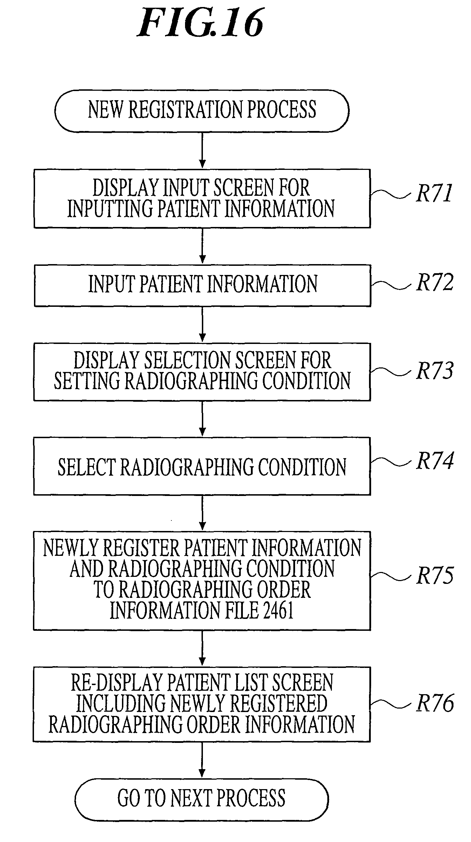

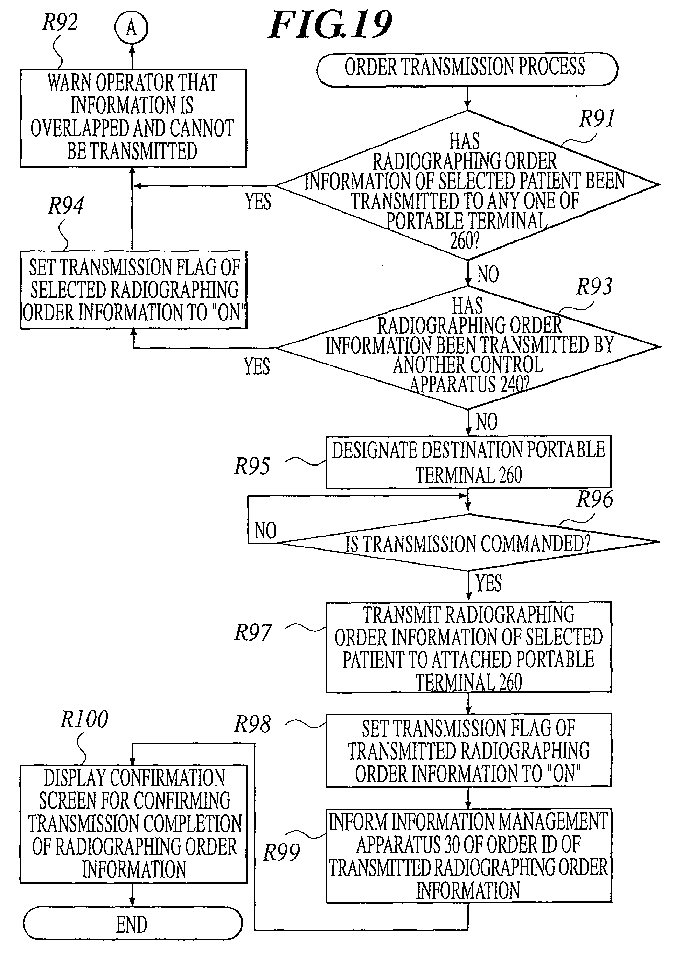

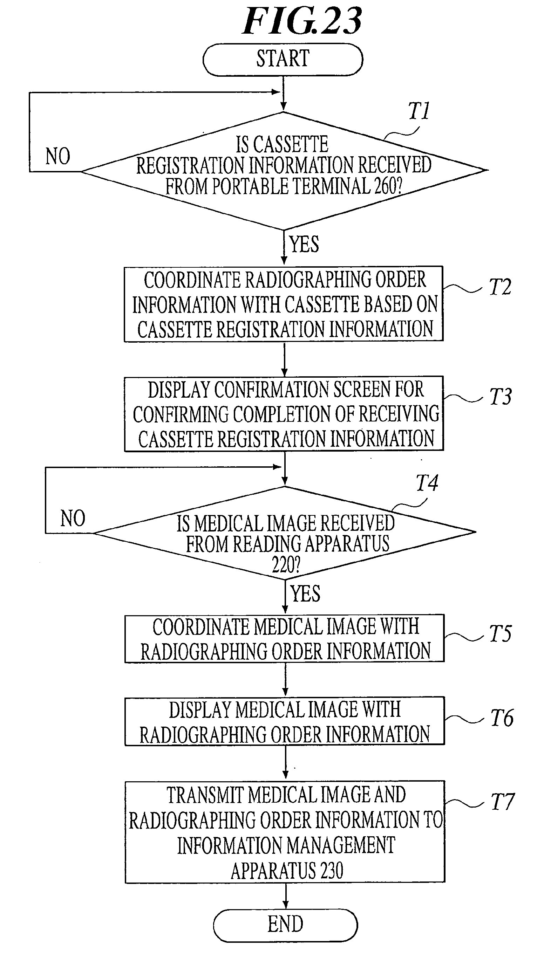

The control unit 241 is equipped with CPU (Central Processing Unit) or the like, develops not only the system program stored in the storage 246, but also a radiography preparation processing program (see FIG. 13), a new registration processing program (see FIG. 16), an order transmission processing program (see FIG. 19), an image registration processing program (see FIG. 23), etc. into RAM 245, and collectively controls the processing operation in cooperation with these programs.

The input unit 242 is equipped with a keyboard having numeric keys, character keys, various kinds of function keys, etc. to input patient information and radiographing information and a touch panel integrally formed with the display unit 243, and outputs the operation signal corresponding to an operated key to the control unit 241.

The display unit 243 is equipped with LCD (Liquid Crystal Display) or the like, and displays various operation screens and various kinds of display information such as processing results of the control unit 241, etc. In the order transmission processing, a warningmessage for warning redundant transmission of radiographing order information is displayed.

The communication unit 244 is equipped with a network interface, a modem or the like, and communicates information with external equipment on the communication network N. For example, it receives radiographing order information from the information management apparatus 230 and also transmits the radiographing order information indicated by a radiographing operator to the portable terminal 260.

RAM 245 forms a work area for temporarily storing various kinds of programs executed by the control unit 241 and data associated with the programs.

The storage 246 is equipped with a magnetic or optical recording medium, or a semiconductor memory, stores not only the system program, but also a radiograph preparation program, a new registration processing program, an order transmission processing program, an image registration processing program and data processed on the basis of the respective programs.

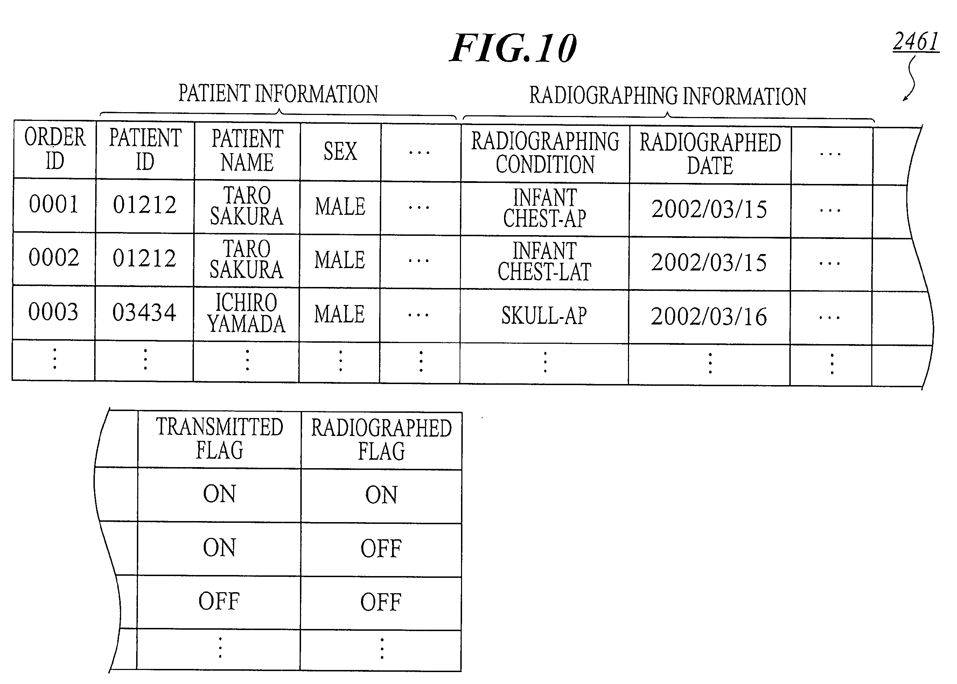

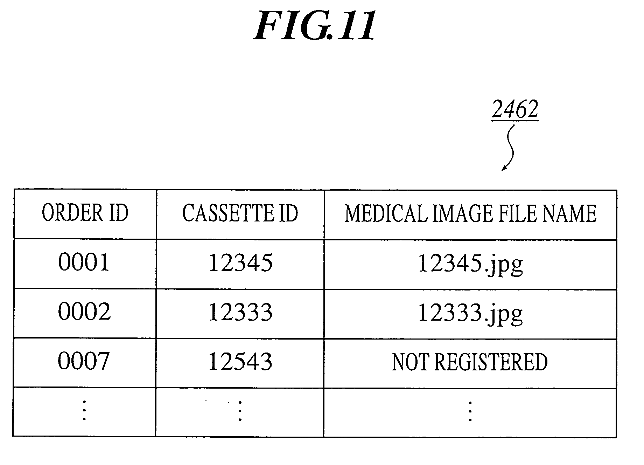

The storage 246 has an image data base (not shown), and stores medical image data received from the reading apparatus 220 into the image data base. It is equipped with a radiographing order information file 2461 (see FIG. 10) for renewably storing the radiographing order information from the information management apparatus 230, and a registered information file 2462 (see FIG. 11) for storing the corresponding relationship between the radiographing order information and the medical image achieved through the radiography on the basis of the radiographing order information.

Radiographing order information is stored in the order of the order ID as shown in FIG. 10. The radiographing order information contains information on a patient such as ID of the patient to be radiographed, the name of the patient, etc. (hereinafter referred to as "patient information"), a radiographing condition, information on radiography such as a radiographing date, etc. (hereinafter referred to as "radiographing information"). In each radiographing order information are set a transmission-completion flag indicating whether transmission of radiographing order information to the portable terminal 260 has been completed, and a radiography-completion flag indicating whether radiography has been completed. The transmission-completion flag is set to "ON" for radiographing order information transmitted to the portable terminal 260, and the transmission-completion flag is set to "OFF" for radiographing order information which has not yet been transmitted. Furthermore, the radiograph-completion flag is set to "ON" for radiographed radiographing order information, and the radiograph-completion flag is set to "OFF" for non-radiographed radiographing order information.

As shown in FIG. 11, in the registered information file 2462 are stored an order ID indicating radiographing order information, a cassette ID indicating a cassette c used for the radiography of the radiographing order information, and the file name of a medical image read out from the cassette c.

Next, the portable terminal 260 will be described.

The portable terminal 260 is a portable type information processing device connectable to the control apparatus 240 through the communication terminal 260a, and it receives and displays radiographing order information based on the portable radiography from the control apparatus 240. The portable terminal 260 establishes the association (correspondence relationship) between the radiographing order information and the cassette c used for the radiography based on the radiographing order information concerned (hereinafter referred to as "cassette registration"), and transmits the correspondence relationship therebetween as cassette registering information to the control apparatus 250.

The communication terminal 260a is connected to the communication network N in a wire communication mode (for example, through a cable or the like), and controls the transmission/reception of information between the portable terminal 260 mounted in the communication terminal 260a and the control apparatus 240. The communication terminal 260a charges the portable terminal 260 mounted therein.

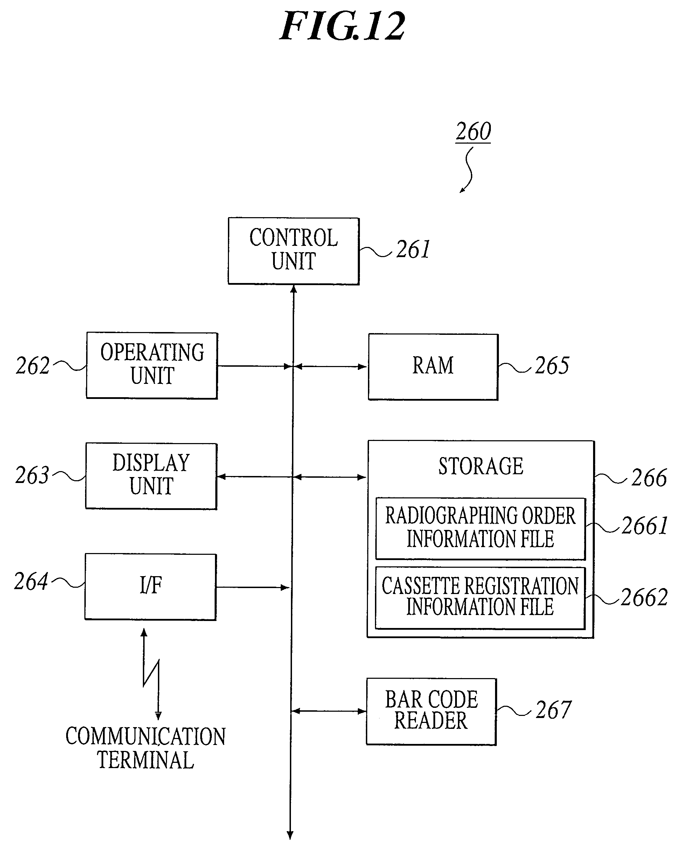

FIG. 12 shows the functional construction of the portable terminal 260.

As shown in FIG. 12, the portable terminal 260 is equipped with a control unit 261, an input unit 262, a display unit 263, an interface (hereinafter referred to as "I/F") 264, RAM 265, a storage 266 and a bar code reader 267.

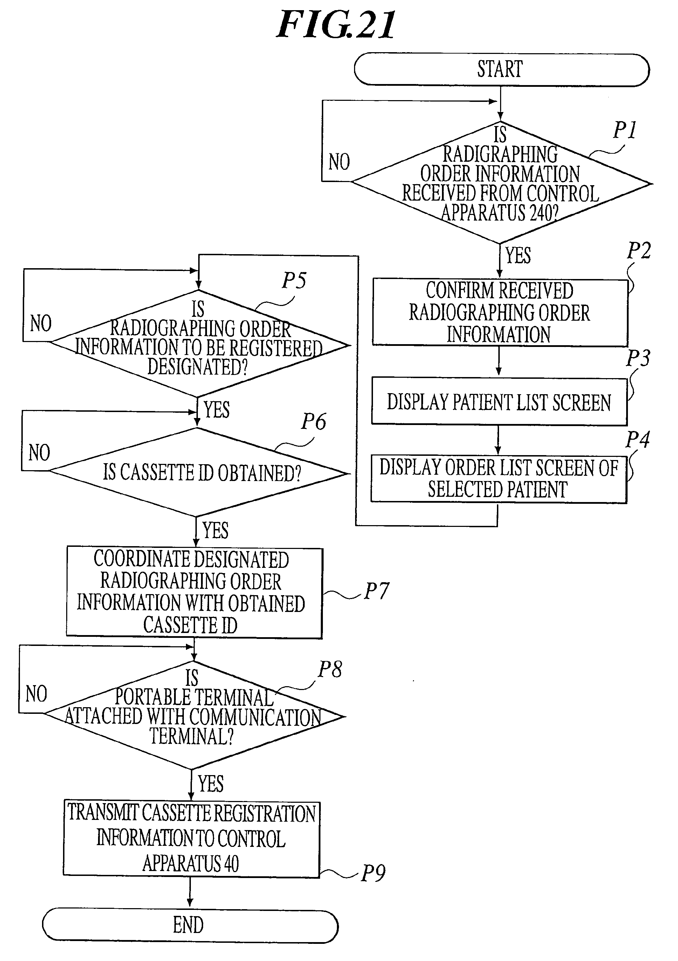

The control unit 261 is equipped with CPU or the like, develops not only the system program stored in the storage 266, but also a cassette registration processing program (see FIG. 21) according to the present invention, etc. into RAM 265, and correctively control the processing operation in cooperation with the programs.

The input unit 262 is equipped with a keyboard having numeric keys, character keys, various function keys, etc., a touch panel formed integrally with the display unit 263, a jog dial or the like.

The display unit 263 is equipped with LCD or the like, and displays various kinds of operation screens, input information from the input unit 262 and various kinds of display information such as the processing results of the control unit 261, etc.

I/F 264 is an interface for connecting the portable terminal 260 and the communication terminal 260a, and it outputs a detection signal to the control unit 261 when the portable terminal 260 is mounted in the communication terminal 260a. Furthermore, I/F 264 receives radiographing order information from the control apparatus 2540 through the communication terminal 260a, and transmits the information on the correspondence relationship between the radiographing order information and the cassette ID of the cassette c used for the radiography based on the radiographing order information as cassette registering information to the control apparatus 240. I/F 264 may connect to a cellular phone terminal such as PHS or the like and establishes wireless communications to transmit/receive data as occasion demands.

RAM 265 forms a work area for temporarily storing various kinds of programs executed by the control unit 261 and data associated with these programs.

The storage 266 is equipped with a magnetic or optical storage medium or semiconductor memory, and stores not only the systemprogram, but also the cassette registrationprocessing program and data processed by the respective programs.

In the storage 266 are stored a radiographing order information file 2661 (not shown) for renewably storing the radiographing order information received from the control apparatus 240, and a cassette registration information file 2662 (not shown) for storing the correspondence relationship between the radiographing order information and the cassette c used for the radiography based on the radiographing order information.

The data structure of the radiographing order information file 2661 is achieved by merely deleting only the item of the transmission-completed flag from the radiographing order information file 2461 equipped in the control apparatus 240, and thus the illustration and description thereof is omitted. Thatis, Pluralradiographingorderinformationpiecesarestored in the order of order-ID, and radiography-completion flags are set.

The data structure of the cassette registration file 2662 is achieved by merely deleting the item of the medical image file name from the registration information file 2462 equipped to the control apparatus 240, and thus the illustration and description thereof are omitted. That is, the order ID representing radiographing order information and the cassette ID used for the radiography based on the radiographing order information concerned are stored in association with each other in the cassette registering information file 2662.

Abar code reader 2 67 has a scanner having an optical reading mechanism, and it reads out the bar code of the cassette ID from the surface of the cassette c, and decodes the bar code thus read according to a predetermined standard to achieve the cassette ID representing the bar code. A patient ID may be affixed in the form of a bar code at patient's bed side or to a part of the patient's body, so that the patient ID is achieved by reading the affixed bar code with the bar code reader 267.

Next, the operation of this embodiment will be described.

First, the radiography preparation processing executed by the control apparatus 240 will be described with FIG. 13. The radiography preparation processing is the processing of transmitting radiographing order information of portable radiography to the portable terminal 260 when the portable radiography is carried out at a doctor's round visit place.

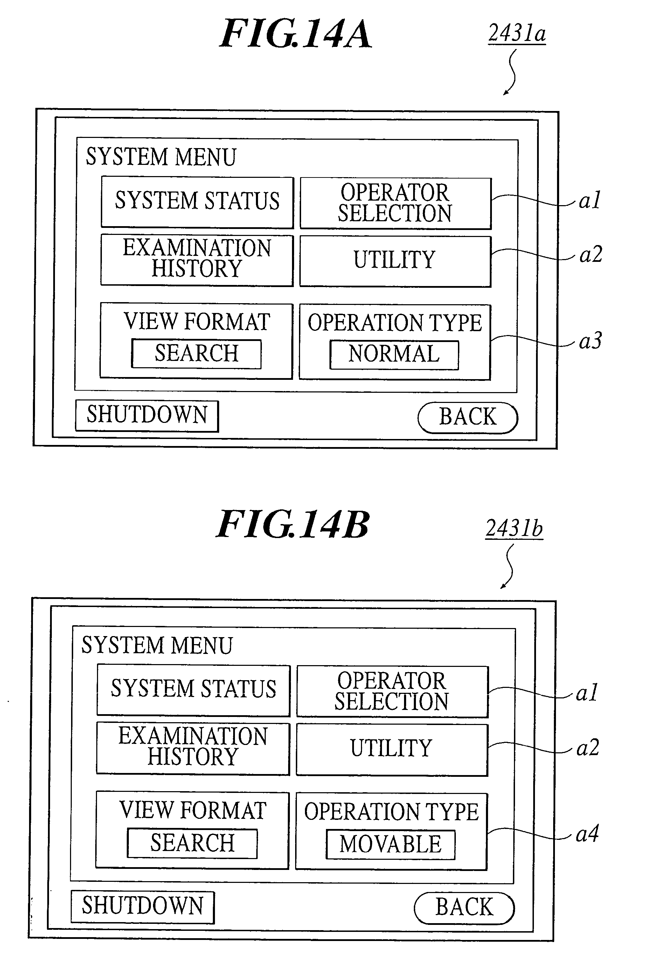

In the radiography preparation processing shown in FIG. 13, in step R1, a guidance is first made to promote a radiographing operator to judge which one of a normal radiographing mode for carrying out radiography by using a radiograph reading apparatus fixed to a radiographing room or a portable radiographing mode for carrying out radiography by using a portable radiographing apparatus 210 using a cassette at a doctor's round visit place should be selected. When the normal radiography is selected (step R1: N), a menu screen 2431a (see FIG. 14A) for normal radiography is displayed on the display unit 243 (step R2), and the processing goes to the processing adapted to the normal radiographing mode.

On the other hand, when the portable radiography is selected (step R1:Y), a menu screen 2431b (see FIG. 14B) for portable radiography is displayed (step R3). As shown in FIG. 14B, a menu key for selecting each of various kinds of menus such as an "operator selection" menu a1 for selecting a radiographing operator, an "operation mode" menu key a2 for shifting to the preparation processing for radiography, etc. is displayed on the menu screen 2431b. When radiography is prepared, the radiographing operator pushes the "operation mode" menu key a2.

When the "operation mode" menu key a2 is pushed on the menu screen 2431b, request information for requesting radiographing order information for portable radiography to the information management apparatus 230 is created, and the request information thus created is transmitted to the information management apparatus 230 by the communication unit 244. When non-radiographed radiographing order information for portable radiography exists in the information management apparatus 230, the radiographing order information concerned is achieved from the information management apparatus 230 (step R4). The radiographing order information thus achieved is stored in the radiographing order information file 2461.

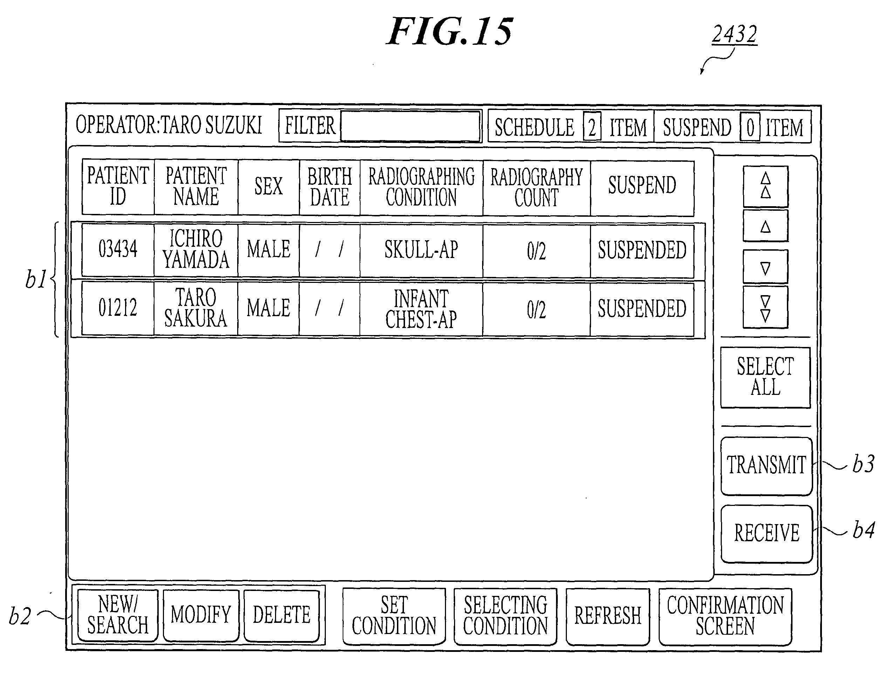

Subsequently, in step R5, a patient list screen on which patients to be radiographed are displayed in the form of a list is displayed on the basis of the radiographing order information thus achieved. At this time, when the radiographing order information is achieved from the information management apparatus 230, a patient list screen 432 on which the patient names of the patients to be radiographed, the patient IDs, etc. are displayed in the form of a list is displayed as shown in FIG. 15. However, when no radiographing order information is achieved from the information management apparatus 230, the patient list screen 432 is displayed while the patient list in the display area b1 of the patient list screen 432 is non-displayed.
When the items of patient ID, patient's name, sex, radiographing condition, the number of radiographs, etc. are displayed in the display area b1 every patient to be radiographed on the patient list screen 432. When a radiographing operator is indicated, the name of the indicated radiographing operator is displayed at the upper left side of the screen. Furthermore, a new registration/search key b2 for making new registration of radiographing order information or searching radiographing order information on the basis of the name of a patient of the patients being displayed is equipped at the lower side of the screen, and a transmission key b3 for instructing transmission of radiographing order information and a reception key b4 for instructing reception of cassette registering information for the radiographing order information of a patient from the portable terminal 260 are equipped at the right side of the screen. When the radiographing operator wants to newly register radiographing order information, the operator pushes the new registration/search key b2. When the operator wants to transmit radiographing order information to the portable terminal 260, the operator selects from the displayed list of the patients a patient whose radiographing order information is wished to be transmitted, puts the portable terminal 260 in the communication terminal 260a and then pushes the transmission key b3 on the patient list screen 2432.

Subsequently, in step R6, it is judged whether new registration of radiographing order information is instructed by the new registration/search key b2. If the new registration of the radiographing order information is instructed (step R6; Y), the processing goes to the new registration processing at the step R7. If new registration of radiographing order information is not instructed (step R6; N), the processing goes to the processing at step R8.

First, the case where the new registration is instructed will be described.

The new registration processing at the step R7 will be described with reference to FIG. 16.

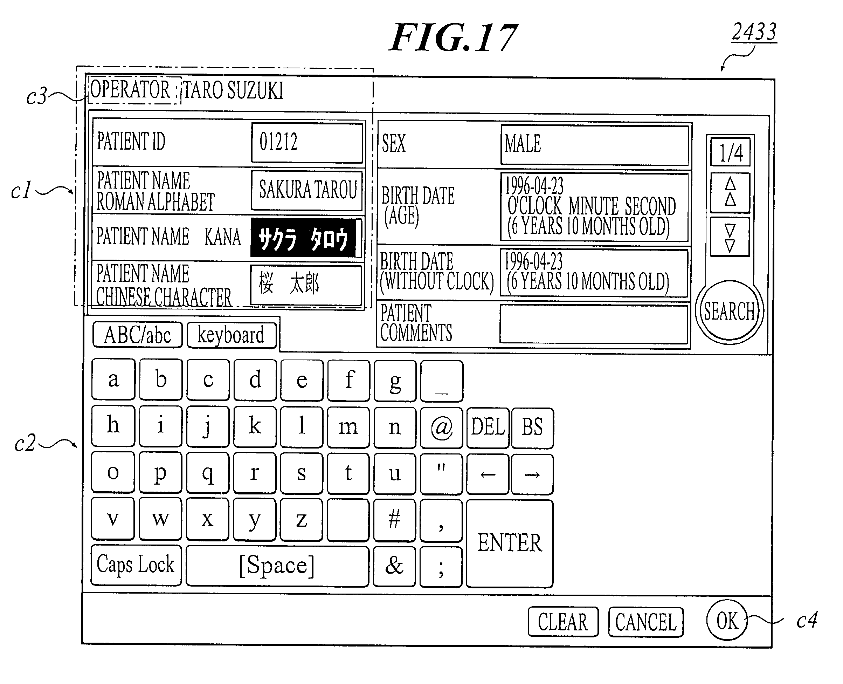

In the new registration processing shown in FIG. 16, an input screen 2433 (see FIG. 17) for inputting patient information is first displayed in step R71. In the input screen 2433 are displayed input areas c1 for inputting various kinds of patient information such as the patient ID, the patient's name (roman letters, Japanese (Kana characters, Chinese characters), English, etc.), sex, birth date, etc., and character keys c2 as shown in FIG. 17.

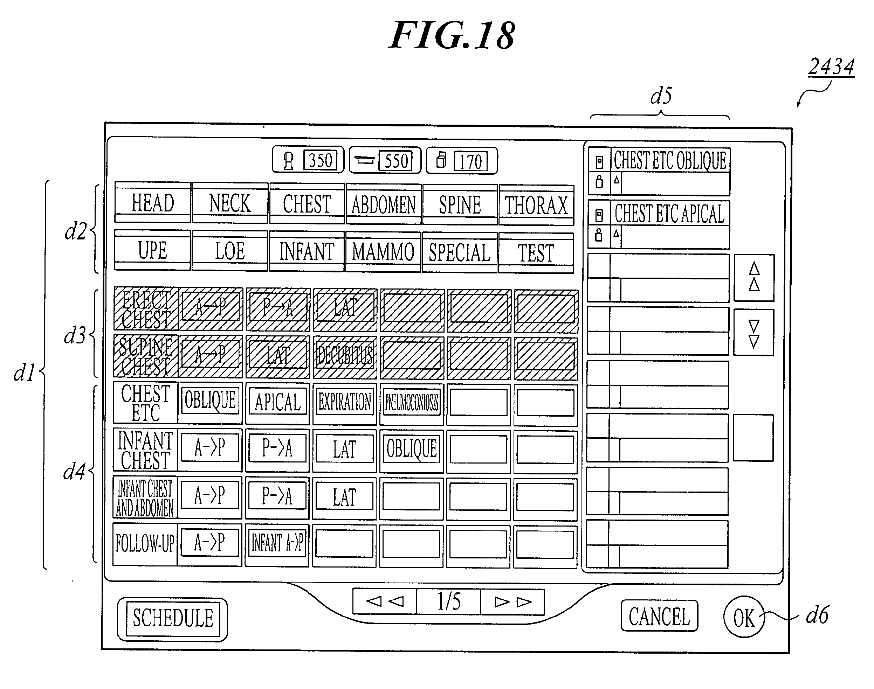

Subsequently, when the radiographing operation inputs patient information into each input area c1 by using the character keys c2 in step R72, a radiographing condition selecting screen 2434 (see FIG. 18) for setting a radiographing condition is displayed in step R73. As shown in FIG. 18, a radiographing condition key group d1 for selecting a radiographic part and a radiographing direction is displayed in the selecting screen 2434. The radiographing condition key group d1 contains keys for selecting radiographic parts and radiographing directions for which radiography in the normal radiographing mode is possible. Of these radiographing condition keys d1, radiographing condition keys d2 for radiographic parts and radiographing directions for which radiography in the portable radiographing mode is impossible are displayed as being hatched so as to indicate that they cannot be selected. In this embodiment, the selection-impossible radiographing condition keys d2 are displayed as being hatched. However, the present invention is not limited to this display mode. For example, the radiographing condition keys d2 may be non-displayed. Alternatively, the radiographing condition keys d2 are not hatched, but displayed in the display mode as the selectable radiographing condition keys d1. In this case, if the radiographing operator erroneously selects a selection-impossible radiographing condition key d2, it may be warned that it is impossible to select the key concerned.

Subsequently, when the radiographing operator selects a radiographing condition by using the radiographing condition keys d1 in step R74, a new order ID is issued to the input patient information and the selected radiographing condition and newly registered in the radiographing order information file 2461 in step R75. Subsequently, in step R76, the patient list screen 432 is displayed again while added with the radiographing order information thus newly registered, and the processing returns to the next processing, that is, the processing of the step R6 of FIG. 13.

Next, the case where the new registration of radiographing order information is not instructed in step R6 will be described.

In step R8, it is judged whether a patient whose radiographing order information is transmitted to the portable terminal 260 is selected from the list of the patients displayed on the patient list screen 432. If the patient whose radiographing order information is transmitted is selected (step R8; Y), the processing goes to the order transmission processing of step R9. If a patient whose radiographing order information is transmitted is not selected (step R8; N), the processing returns to the step R6.

The order transmission processing of the step R9 will be described with reference to FIG. 19.

In the order transmission processing shown in FIG. 19, it is judged in step R91 whether the radiographing order information of the selected patient has been already transmitted to some portable terminal 260. This judgment is made on the basis of a judgment as to whether a transmission-completion flag is set to "ON" in the radiographing order information file 2461.

When the transmission-completion flag of the radiographing order information of the selected patient is set to "ON" and thus it is judged that the radiographing order information has been already transmitted to the portable terminal 260 (step R91; Y), a message warning that the radiographing order information has been already transmitted to some portable terminal 260 and thus it cannot be redundantly transmitted is displayed on the display unit 243, or an alarm sound warning the foregoing fact is output (step R92). After the warning, the processing goes to the processing of the step R8 of FIG. 13.

On the other hand, when the transmission-completion flag of the radiographing order information of the selected patient is set to "OFF" and thus it is judged that the radiographing order information has not yet been transmitted to any portable terminal 260 (step R91; N), the order ID of the radiographing order information of the selected patient is transmitted to the information management apparatus 230, and inquiry information for inquiring bout whether the radiographing order information concerned has been transmitted from another control apparatus 240 to some portable terminal 260 is transmitted to the information management apparatus 230. On the basis of the response information received from the information management apparatus 230, it is judged whether the radiographing order information of the selected patient has been already transmitted from another control apparatus 240 to some portable terminal 260 (step R93).

If it is judged that the radiographing order information of the selectedpatient has been already transmitted from another control apparatus 240 to some portable terminal 260 (step R93; Y), the transmission-completion flag of the radiographing order information of the selected patient is set to "ON" in the radiographing order information file 2461 (step R94), and the processing goes to the step R92 to warn that the radiographing order information concerned has been already transmitted to some portable terminal 260 and thus it cannot be redundantly transmitted. After the warning, the processing goes to the step R8 of FIG. 13.

On the other hand, if the selected radiographing order information has not yet been transmitted (step R93;N), in step R95 connectable portable terminals 260 are displayed on the display unit 243 so as to be selectable, and a guidance is made to promote the operator to indicate a portable terminal 260 to which the radiographing order information is transmitted. When the operator indicates the portable terminal 260 as a transmission destination, it is judged whether a transmission instruction is input by the transmission key a3 (step R96).

When the transmission instruction is input (step R96; Y), the radiographing order information of the patient selected by the indicated portable terminal 260 is transmitted by the communication unit 244 (step R97). At this time, a transmission destination of the cassette registering information for the radiographing order information to be transmitted is indicated, and the information on the indicated transmission destination may be transmitted as accompanying information of the radiographing order information to the portable terminal 260 together with the radiographing order information. After the transmission is finished, in step R98, the transmission-completion flag of the radiographing order information transmitted to the portable terminal 260 is set to "ON" in the radiographing order information file 2461.

Subsequently, in step R99, the order ID of the radiographing order information for which the transmission-completion flag is set to "ON" is transmitted to the information management apparatus 230, thereby notifying that the radiographing order information concerned has been already transmitted. In the information management apparatus 230, the radiographing order information corresponding to the order ID transmitted from the control apparatus 30 is managed as transmission-completed radiographing order information.

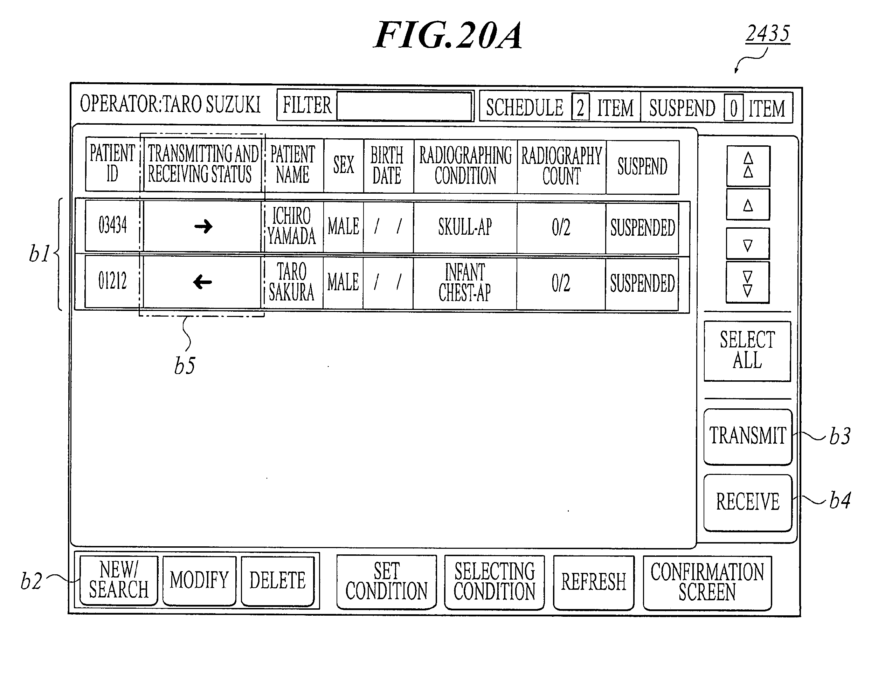

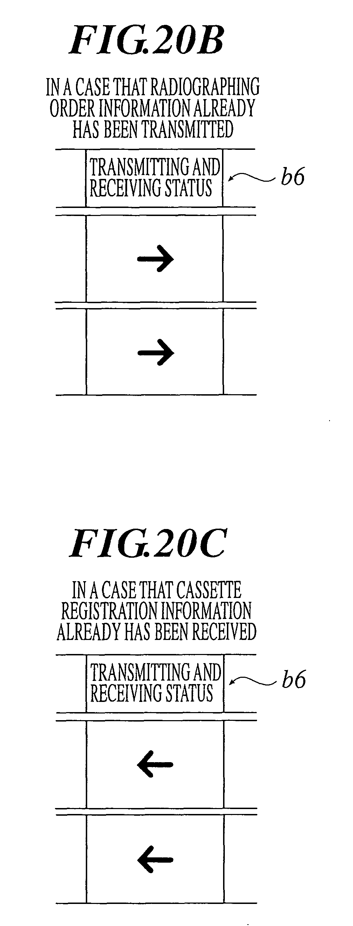

Subsequently, in step R100, a check screen 2435 (see FIG. 20a) for checking transmission completion of radiographing order information is displayed on the display unit 243, and this processing is finished. As shown in FIG. 20A, a transmission/reception status b5 is provided on the check screen 2435 every patient of the patient display list, and as shown in FIG. 20B, a mark "→" representing transmission completion is displayed in the item of the transmission/reception status of a patient (in the displayed patient list) whose radiographing order information is transmitted to the portable terminal 260. On the other hand, when the cassette registering information is received from the portable terminal 260, a mark "←" representing reception completion is displayed in the transmission/reception status b5 of the patient as shown in FIG. 20C.

When the radiographing operator checks that the radiographing order information is transmitted to the portable terminal 260 on the check screen 2435, the operator carries the cassette c, the portable radiographing apparatus 210 to the patient to be radiographed while carrying the portable terminal 260, and prepares for radiography.

Next, the cassette registration processing executed by the portable terminal 260 will be described with reference to FIG. 21. The cassette registrationprocessing serves to associate the radiographing order information transmitted from the control apparatus 240 with the cassette c used for the radiography based on the radiographing order information concerned.

In the cassette registration processing shown in FIG. 21, it is judged in step P1 whether radiographing order information is received from the control apparatus 240. If no radiographing order information is received (step P1; N), the portable terminal 260 waits for reception.

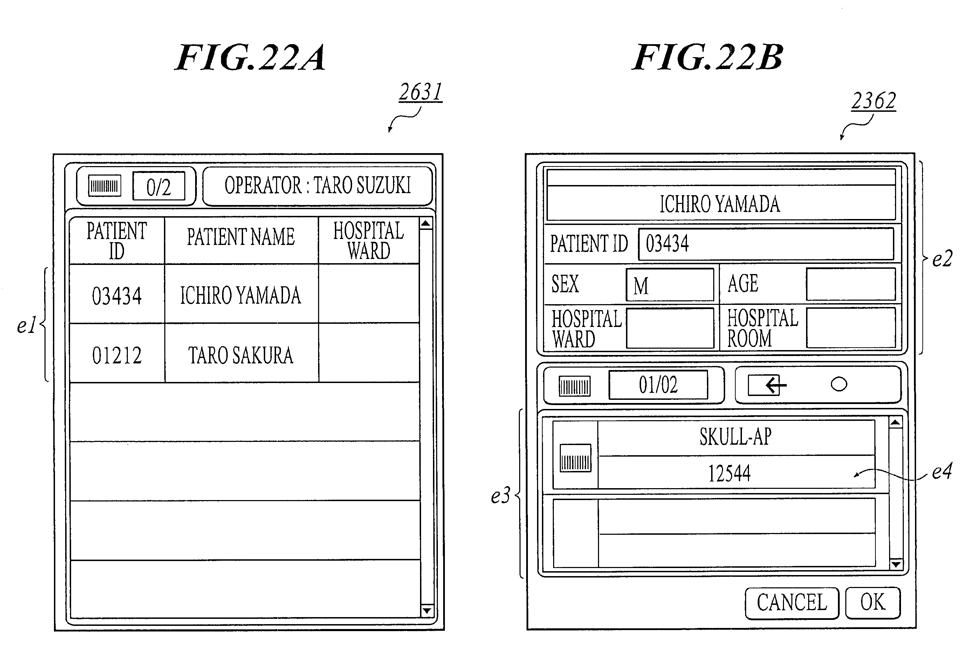

When radiographing order information is received from the control apparatus 240 (step P1; Y), the radiographing order information thus received is stored in the radiographing order information file 2661 (step P2), and in step P3 a patient screen 2631 (see FIG. 22A) is displayed on the display unit 263 on the basis of the radiographing order information thus stored. As shown in FIG. 22A, a list of patient ID, patient's name, etc. of a patient to be radiographed is displayed in a display area e1. Radiographing order information which is out of the display in the display area can be displayed by scrolling the screen. The operator checks the patient list screen 2631 displayed on the portable terminal 260 at the bed side of the patient to be radiographed, and selects and inputs the patient to be radiographed from the displayed patient list.

When the operator selects the patient to be radiographed from the displayed patient list on the patient list screen 2631, an order list screen 2632 (see FIG. 22B) is displayed in step P4, so that the patient information e2 of the selected patient and the radiographing conditions e3 thereof are displayed as a list.

From the list of the radiographing conditions e3 displayed on the order list screen 2632, the operator selects and indicates through a cursor a radiographing condition e3 of radiographing order information for which the operator wants cassette registration, and makes the bar code reader 267 of the portable terminal 260 read the bar code of the cassette ID of the cassette c used for radiography.

At the portable terminal 260, when the radiographing condition e3 of the radiographing order information to be registered is indicated on the order list screen 2632 by the operator (step P5; Y), the bar code of the cassette ID is read out by the bar code reader 267, and it is judged whether the cassette ID is achieved or not (step P6). If no cassette ID is achieved (step P6; N), the processing waits for achievement.

If the cassette ID is achieved (step P6; Y), the indicated radiographing order information and the achieved cassette ID are stored in association with each other in the cassette registering information file 2662 (step P7). After the registration is finished, the cassette IDe4 of the cassette c registered in the radiographing condition e3 is displayed on the order list screen 2632 as shown in FIG. 22B to notify that the cassette registration is completed.

The radiographing operator confirms on the order list screen 2632 that the cassette registration is finished, and radiographs the patient by using the registered cassette c. When the operator radiographs plural patients at doctor' s round visit places, the processing of the steps P2 to P7 is repeated, and the cassette registering information is successively stored in the cassette registering information file 2662. The cassette registration may be carried out by the portable terminal 260 after the radiographing operation irrespective of the cassette registration and radiographing order. After the radiographing operation is carried out, the radiographing operator returns to the radiographing room and mounts the portable terminal 260 in the communication terminal 260a connected to the control apparatus 240.

In the portable terminal 260, it is judged in step P8 whether the portable terminal 260 is mounted in the communication terminal 260a. If the portable terminal 260 is not mounted in the communication terminal 260a (step P8; N), the mounting of the portable terminal 260 is awaited. When the portable terminal 260 is mounted in the communication terminal 260a (step P8; Y) , if a transmission destination of the cassette registering information is indicated in the accompanying information of the radiographing order information, the cassette registering information is transmitted to the control apparatus 240 serving as the indicated transmission destination. If no transmission destination is particularly indicated, the cassette registering information is transmitted to the control apparatus 240 serving as the transmission source of the radiographing order information (step P9), and the processing is finished.

The radiographing operator mounts the portable terminal 250 in the communication terminal 260a, and transmits the cassette registering information to the control apparatus 240. Thereafter, the operator mounts the cassette c in the reading apparatus 220, and operates the control apparatus 24 to instruct reading of a medical image recorded in the cassette c. In the reading apparatus 220, the bar code of a cassette ID is red out from the cassette c mounted, and also the medical image is read out. The cassette ID of the cassette c in which the medical image is recorded in the header area of the read-out medical image is written. The medical image having the cassette ID written therein is transmitted to the control apparatus 240.

Next, the image registration processing executed by the control apparatus 240 will be described with reference to FIG. 23.

This image registration processing associates radiographing order information with a radiographic image transmitted from the reading apparatus 220 on the basis of cassette registering information transmitted from the portable terminal 260.

In the image registration processing shown in FIG. 23, it is judged in step T1 whether cassette registering information is received from the portable terminal 260. If no cassette registering information is received (step T1; N), the reception is awaited. If any cassette registering information is received (step T1; Y), the radiography-completion flag of the radiographing order information for which cassette registration is carried out is set to "ON" in the radiographing order information file 2461, and also the radiographing order information and the cassette ID of the cassette c used for the radiography based on the radiographing order information concerned are stored in association with each other in the registering information file 2462 (step T2).

Subsequently, in step T3, the check screen 2435 (see FIG. 20A) is displayed on the display unit 243 to check whether the cassette registering information corresponding to the radiographing order information transmitted from the control apparatus 240 to the portable terminal 260 is received from the portable terminal 260. As shown in FIG. 20A, a mark "←" representing that the cassette registering information corresponding to the radiographing order information has been already received is displayed in the transmission/reception status b5 on the check screen 2435.

Subsequently, it is judged in step T4 whether the medical image read out from the reading apparatus 220 is received. If the medical image is not received (step T4; N), the reception is awaited. If the medical image is received (step T4; Y), the cassette ID is read out from the header area of the medical image thus received, and the file name of the medical image is stored in association with the cassette ID coincident with the read-out cassette ID in the registering information file 2462 (step T5).

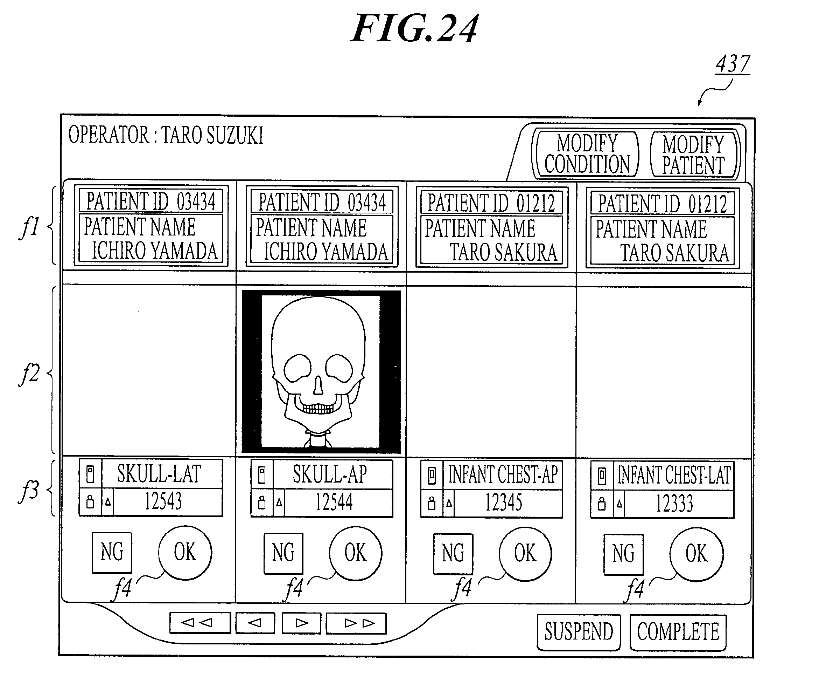

After the registration is finished, an image check screen 2437 (see FIG. 24) on which the radiographing order information and the medical image achieved through the radiography carried out according to the radiographing order information are displayed in association with each other is displayed on the display unit 243 in step T6. As shown in FIG. 24, medical images f2 of plural patients are displayed in a line on the image check screen 2437, and each medical image f2 is displayed in association with patient information f1 containing ID of patient and the name of the patient, and radiographing information f3 containing a radiographing condition and the cassette ID of a cassette used for the radiography based on the radiographing condition. When the cassette registration has been carried out, however, no medical image has been read out, only the patient information f1 and the radiographing information f3 are displayed, and no image is displayed on the medical image f2. The radiographing operator checks the correspondence relationship between the displayedmedical image and the radiographing order information, and if they correspond to each other, the operator pushes an OK key f4.

When the OK key f4 is pushed on the image check screen 2437, in step T7, the data file of the medical image for which the OK key f4 is pushed is transmitted to the image management apparatus 230, and also the correspondence relationship between the medical image and the radiographing order information is read out from the registering information file 2462, and transmitted as image registering information to the image management apparatus 230, thereafter finishing this processing.

In the information management apparatus 230, the medical image and the image registering information are received from the control apparatus, and the medical image is managed in the form of a data base on the basis of the image registering information thus received.

As described above, according to the medical image photographing system 200 of this embodiment, plural portable terminals 260 are connected to a communication network, and radiographing order information for portable radiography is transmitted from a control apparatus 240 to a portable terminal 260 and displayed on the portable terminal 260. Therefore, even when a patient is subjected to radiography at bed side or the like, a radiographing operator can check radiographing order information through a portable terminal 260 carried by the operator, and thus the operation can easily check the patient to be radiographed and the radiographing condition of radiography for the patient. Accordingly, the radiography for achieving medical images at a doctor' s round visit place can be efficiently and accurately performed.

Furthermore, when the cassette ID used for radiography is read out in the portable terminal 260, the cassette registration is carried out and the cassette registering information is transmitted from the portable terminal 260 to the control apparatus 240, the association between the medical image achieved through the radiography and the radiographing order information is carried out on the basis of the cassette registering information in the control apparatus 240. Therefore, the operators work of inputting the correspondence relationship between the radiographing order information and the medical image to the control apparatus 240 can be omitted, and thus the working load on the radiographing operation can be reduced. Furthermore, a human error such as input error or the like by a radiographing operation can be prevented and thus the medical images can be accurately managed.

With respect to radiographing order information transmitted from the control apparatus 240 to any one of the portable terminals 260, the transmission-flag of the radiographing order information concerned is set to "ON", and the radiographing order information for which the transmission-completion flag is set to "ON" is prohibited from being transmitted to another portable terminal 260. Therefore, the same radiographing order information can be prevented from being redundantly transmitted to plural portable terminals.

Furthermore, in the information management apparatus 230, the transmission status of the radiographing order information which is simultaneously transmitted to plural control apparatuses 240 is managed, and radiographing order information which has been already transmitted from a control apparatus 240 to a portable terminal 260 is prohibited from being transmitted from another control apparatus 240 to a portable terminal 260, so that the same radiographing order information can be prevented from being redundantlytransmitted to pluralportableterminals.

Still furthermore, when a radiographing operator indicates radiographing order information having the "ON"-set transmission-completion flag as radiographing order information to be transmitted to the portable terminal 260 at the transmission time of the radiographing order information, it is warned that the radiographing order information cannot be transmitted because it has been already transmitted (i.e., transmission is completed) . Therefore, operator's attention can be attracted to the fact that the radiographing order information has been already transmitted, and thus the radiographing order information can be prevented from being redundantly transmitted by mistake.

As described above, by preventing the redundant transmission of the same radiographing order information, plural radiographing operators can be prevented from making preparations for radiography at the same time, and also redundant radiography can be prevented from being carried out on the same patient, so that the radiography at a doctor' s round visit place can be accurately performed.

According to the system construction described above, the portable terminal 260 can connect to any control apparatus 240, and thus radiographing order information can be transmitted from any control apparatus 240 to a desired portable terminal 260, thereby enhancing convenience.

The foregoing description of this embodiment relates to apreferableembodimentofthemedicalimagephotographingsystem to which the present invention is applied, and the present invention is not limited to this embodiment.

For example, in the foregoing description, when radiographing order information indicatedby a portable terminal 260 is transmitted from a control apparatus 240, it is inquired to the information management apparatus 230 whether the radiographing order information concerned has been already transmitted from another control apparatus. However, the present invention is not limited to this embodiment. For example, when it is notified from each control apparatus 240 to the information management apparatus 230 that some radiographing order information has been already transmitted, the order ID of the transmitted radiographing order information concerned may be distributed to each control apparatus 240 to notify that the radiographing order information has been already transmitted from another control apparatus. Accordingly, the transmission status of radiographing order information can be grasped on a real-time basis at each control apparatus 240.

With respect to the detailed construction and detailed operation of the medical image photographing system according to this embodiment, they may be properly modified without departing from the subject matter of the present invention.

According to the present invention, the radiographing order information is displayed on the portable terminal, so that even when radiography is carried out at a doctor's round visit place such as a patient bed side or the like, a radiographing operator can easily check a patient to be radiographed and a radiographing condition by using a portable terminal. Accordingly, the radiography at the doctor' s round visit place can be efficiently performed. Furthermore, radiographing order information transmitted from a control apparatus to a portable terminal is prohibited f rombeing transmitted to another portable terminal, so that the same radiographing order information can be prevented from being redundantly transmitted to plural portable terminals. Accordingly, radiography can be prevented from being redundantly carried out on the same patient, and the radiography at the doctor' s round visit place can be accurately performed.

Furthermore, radiographing order information transmitted from a control apparatus to any portable terminal can be prohibited from being transmitted from another control apparatus, so that the same radiographing order information can be prevented frombeing redundantly transmitted to plural portable terminals. Accordingly, the redundant radiography based on the same radiographing order information can be prevented, and the radiography at the doctor' s round visit place can be accurately performed.

Still furthermore, it is warned that transmission to some portable terminal has been completed, so that operator's attention can be attracted to the fact that transmission has been completed.

### [Third Embodiment]

In the first and second embodiments, the description has been made to the medical image photographing system in which portable terminals and control apparatuses are connected to one another in a connection mode of M:N. In the third embodiment, the description will be made to a medical image photographing system in which portable terminals and control apparatuses are connected to one another in a master-slave connection mode, and a radiographing condition and a cassette can be easily associated with each other by using a portable terminal in such a connection mode that a portable terminal can transmit/receive data to/from specific control apparatuses.

The third embodiment to which the present invention is applied will be described in detail with reference to the accompanying drawings.

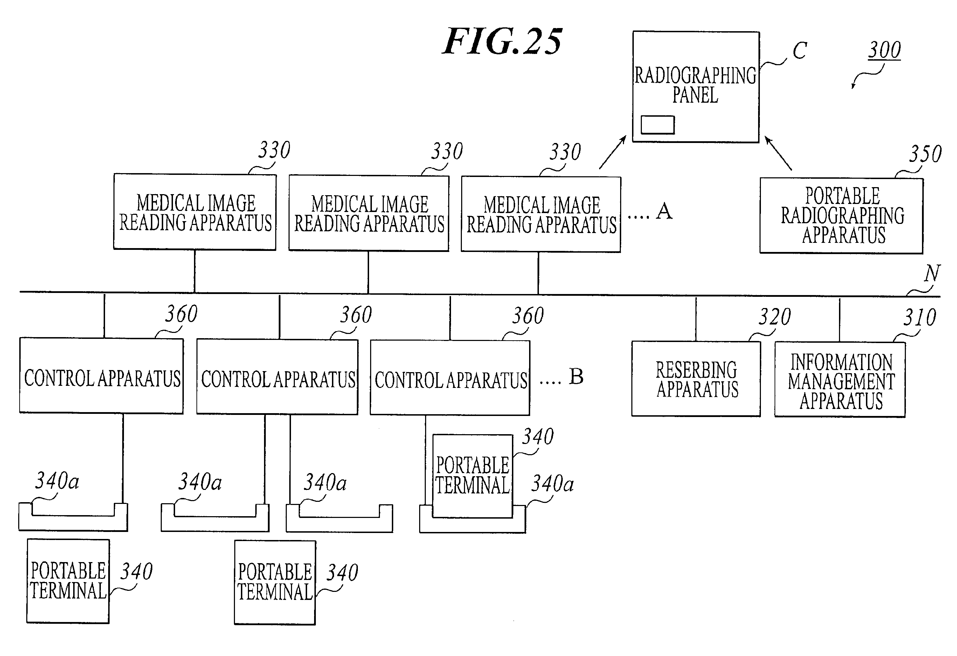

First, the schematic construction of a medical image photographing system 300 will be first described with reference to FIG. 25. FIG. 25 is a block diagram showing the schematic construction of the medical image photographing system 300.

As shown in FIG. 25, substantially as in the case of the medical image photographing system 100 according to the first embodiment, the medical image photographing system set up in hospital facilities is equipped with an information management apparatus 310, a reserving apparatus 320, medical image reading apparatuses 330, portable terminals 340, communication terminals 340a, a portable radiographing apparatus 350, control apparatuses 360, etc.

The information management apparatus 310, the reserving apparatus 320, the medical image reading apparatuses 330 and the control apparatuses 360 can mutually transmit/receive data to/from one another through a network N such as LAN (Local Area Network), WAN (Wide Area Network) or the like which is constructed in the hospital facilities.

One or plural communication terminals 340a are connected to each control apparatus 360 in a wire communication mode (i. e., through a cable) in advance to transmit/receive data to/from each portable terminal 340. The setup numbers of the medical image reading apparatuses 330 and the control apparatuses 360 may be set to any numbers. For example, it is assumed that medical image reading apparatuses 330 of A and control apparatuses 360 of B are setup. Each of the control apparatuses 360 can communicate with all the medical image reading apparatuses 330 of A through the network N, and achieve radiographic image data from any medical image reading apparatus 330.

With respect to the information management apparatus 310, the reserving apparatus 320 and the portable radiographing apparatus 350, a plurality of apparatuses can be also set up in the hospital facilities. Particularly, the portable radiographing apparatuses 350 may be disposed at each dedicated space or nurse station (not shown) equipped on each floor in the hospital facilities.

When receiving radiographing order information transmitted from HIS/RIS or the control apparatus 360, the information management apparatus 310 stores the radiographing order information thus received.

Here, the radiographing order information is beforehand added with a flag (hereinafter referred to as "portable indication flag") indicating which one of the normal radiographing mode and the portable radiographing mode the radiographing order information concerned is based on. Here, the normal radiographing mode is defined as a mode in which radiography will be carried out in a radiographing room, and the portable radiographing mode is defined as a mode in which radiography will be carried out at bed side in a hospital room by using the portable radiographing apparatus 350. When the portable indication flag is set to "ON", it means the radiographing order information for portable radiography, and when the portable indication flag is set to "OFF", it means the radiographing order information for radiography in the radiographing room.

Upon receiving radiographing order information added with panel identification information transmitted from the control apparatus 360, the information management apparatus 310 renews the radiographing order information stored in advance to the radiographing order information added with the panel identification information thus received. The information management apparatus 310 stores the radiographic image data achieved from the medical image reading apparatus 330.

Here, the construction of the information management apparatus 310 and the radiographing order information added with the panel identification information will be described in detail later.

The reserving apparatus 320 is equipped to the diagnosis accept room or each medical examination room, and it is an input terminal for inputting radiographing order information relating to radiography by a doctor or the like. PC terminal equipped to each medical examination room or the diagnosis accept room corresponds to the reserving apparatus 320. The reserving apparatus 320 transmits the input radiographing order information to HIS/RIS.

The medical image reading apparatus 330 reads out radiographic image data from a radiographing panel C set in a reading unit (not shown). Here, the radiographing panel is an imaging plate having a photostimulable phosphor layer formed thereon or a cassette having the imaging plate (all the elements are not shown). The medical image reading apparatus 330 emits fluorescence corresponding to accumulative radiation energy from the photostimulable phosphor layer, photoelectrically converts the fluorescence thus emitted to electrical signals and outputs the electrical signals as radiographic image data.

Abar code inherent to the radiographing panel C is attached as panel identification information on the surface of the radiographing panel C, that is, the imaging plate and/or the cassette. The panel identification information of the radiographing panel c is not limited to a specific material insofar as the radiographing panel C is identifiable on the basis of the panel identification information. For example, an IC chip in which panel identification information is recorded may be used.

The portable terminal 340 is a portable type information terminal carried by a radiographing operator who operates the portable radiographing apparatus 350 when radiography is carried out. The portable terminal 340 is set up together with the communication terminal 340a, the radiographing panel C, the portable radiographing apparatus 350, etc. at a predetermined place in a hospital ward, for example, at a nurse station or the like.

When a portable terminal 340 is set in a communication terminal 340 and allowed to make data communications with a control apparatus 360 connected to the communication terminal 340a concerned through a cable, the portable terminal 340 transmits/receives data to/from the control apparatus 360 concerned. The portable terminal 340 can transmit/receive data to/from only a specific control apparatus 360 which is connected to the communication terminal 340a through a cable in advance.

The portable terminal 340 is equipped with PDA (Personal Digital Assistant), for example, however, it may be a laptop PC or the like. The communication terminal 340a is a cradle, for example.

Here, the construction of the portable terminal 340 will be described in detail layer.
The portable radiographing apparatus 350 is a radiographing apparatus movable between hospital rooms or beds, and carries out radiography (X-ray photographing) at bed side in each hospital room by using the radiographing panel C.

The control apparatus 360 transmits/receives data with the portable terminal (s) 340 through one or plural communication terminals 340a which are connected to the control apparatus 360 concerned through a cable in advance. That is, the control apparatus 360 transmits/receives data to/from only a specific portable terminal (s) 340 through the communication terminal (s) 340a.

The control apparatus 360 downloads radiographing order information from the information management apparatus 310, and transmits the radiographing order information thus downloaded to each portable terminal 340 through the communication terminal 340a which is connected thereto through a cable in advance. Furthermore, the control apparatus 360 can input and edit radiographing order information, and uploads to the information management apparatus 310 the radiographing order information thus input/edited or the radiographing order information added with panel identification information transmitted from the portable terminal 340.

The control apparatus 360 can display radiographic image data achieved from the medical image reading apparatus 330, so that the operator can easily check the radiography result. In this case, the medical image reading apparatus 330 and the control apparatus 360 are preferably set up in the same room or on the same floor, so that the operator can check the radiography result without moving between rooms or floors.

Here, the construction of the control apparatus will be described in detail later.

That is, when the radiographing order information input from HIS/RIS or the control apparatus 360 is transmitted to the portable terminal 340 in the hospital ward, the radiographing operator carries the portable terminal 340 concerned and refers to the radiographing order information displayed on the screen of the portable terminal 340 to check a patient to be radiographed at bet side of the hospital room and the size/number of required radiographing panels C. Subsequently, the radiographing operator carries the radiographing panels C whose size and number are checked, the portable terminal 340 and the portable radiographing apparatus 350 to the bed side of a hospital room in which the patient to be radiographed is accommodated. The radiographing operator records the association (correspondence relationship) between the radiographing order information and the radiographing panel C (that is, the panel identification information) into the portable terminal 340 every radiographing operation, and the portable terminal 340 is set in the communication terminal 340a after all the radiographing operations are finished. At this time, the radiographing order information added with the panel identification information is automatically uploaded from the portable terminal 340 to the information management apparatus 310.

The portable terminal 340 may have a function of collating the patient ID input by the radiographing operator with the patient ID contained in the radiographing order information, and then displaying the collation result on the screen of the display unit 343 or notifying the collation result with sound. Furthermore, it is preferable to input the patient ID to the portable terminal 340 by using a bar code. In this case, the work of checking the patient to be radiographed is not carried out by operator's visual observation, but also carried out by a computer, so that reliability is enhanced.

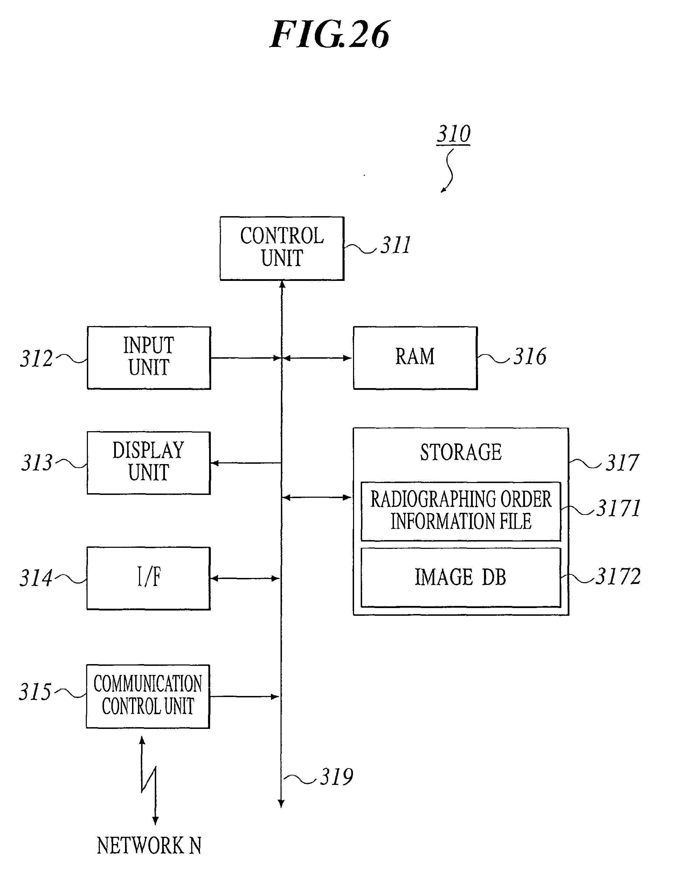

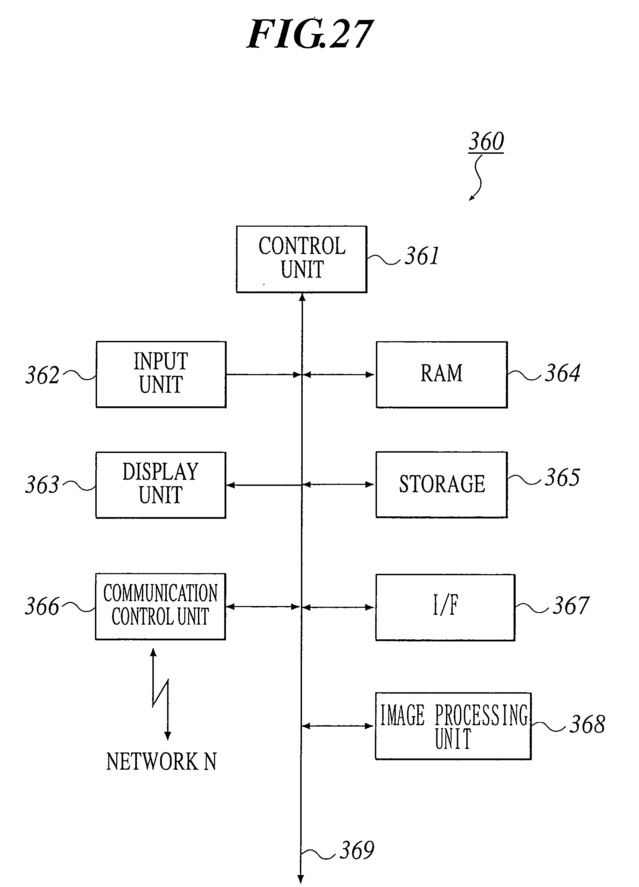

Next, the details of the construction of each of the information management apparatus 310 and the control apparatus 360 will be described with reference to FIGS. 26 and 27. The internal construction of the portable terminal is substantially the same as the portable terminal 140 of the first embodiment shown in FIG. 3, and thus the construction of the portable terminal 340 will be described in detail with reference to FIG. 3. FIG. 26 shows the internal construction of the information management apparatus 310, and FIG. 27 shows the internal construction of the control apparatus 360a.

First, the construction of the information management apparatus 310 will be described.

As shown in FIG. 26, the information management apparatus 310 is equipped with a control unit 311, an input unit 312, a display unit 313, I/F 314, a communication control unit 315, RAM 316, a storage 317, etc. Theses elements are connected to one another through a bus 319.

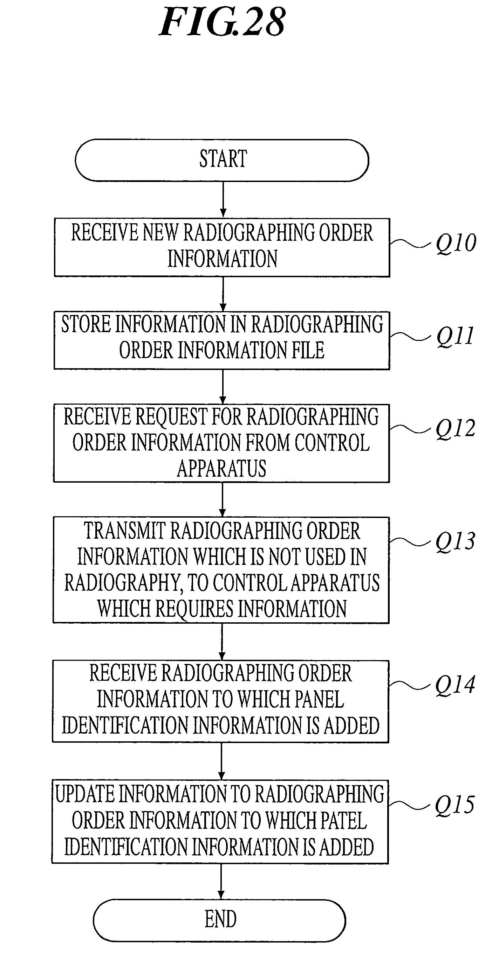

The control unit 311 reads and executes various kinds of programs stored in the storage 317. Particularly, the control unit 311 reads out from the storage unit 317 the program for executing the information processing represented by the flowchart of FIG. 28 described in detail later, and executes the program thus read out.

Upon receiving radiographing information transmitted from HIS/RIS or the control apparatus 360, the control unit 311 records the radiographing order information concerned into a radiographing order information file 3171. When receiving radiographing order information added with panel identification information transmitted from the control apparatus 360, the control unit 311 renews the radiographing order information recorded in the radiographing order information file 3171 in advance to the radiographing order information added with the received panel identification information. Furthermore, the control unit 311 stores radiographic image data achieved from the medical image reading apparatus 330 into an image DB (Data Base) 3172.

The input unit 312, the display unit 313, the communication control unit 315 and RAM 316 have substantially the same construction and operation as the input unit 112, the display unit 113, the communication control unit 115 and RAM 116 of the first embodiment, and the detailed description thereof is omitted.

I/F 314 is an interface for transmitting/receiving data to/from the control apparatus 360, and receiving radiographing order information from HIS or RIH.

The storage 317 is equipped with a freelywritable/erasable non-volatile semiconductor memory such as HDD (Hard Disc Drive) or the like, and stores various kinds of programs and various kinds of data. Particularly, the storage 317 stores the program for executing the information processing represented by the flowchart of FIG. 28 described in detail later.

The storage 317 has the radiographing order information file 3171 and the image DB 3172. The radiographing order information file 3171 records radiographing order information input from HIS/RIS or the control apparatus 360, and the image DB 3172 stores, in association with patient ID, radiographic image data which have been subjected to the image processing by the control apparatus 360.

The storage unit 317 maybe equipped with a recording medium (not shown) in which various kinds of programs and various kinds of data are stored in advance. The recording medium is a non-volatile memory which is fixedly or freely detachably equipped to the storage 317 and formed of a magnetic recording medium, an optical recording medium or a semiconductor memory.

Next, the construction of the portable terminal 340 will be described.

The portable terminal 340 is equipped with a control unit 341, an input unit 342, a display unit 343, a reading unit 344, RAM 345, storage 346, I/F 347, etc., and these elements are connected to one another through a bus 349. The respective parts of the portable terminal 340, that is, the control unit 341, the input unit 342, the display unit 343, the reading unit 344, RAM 345, the storage 346, I/F 347 and the bus 349 correspond to the respective parts of the portable terminal 140 of the first embodiment, that is, the control unit 141, the input unit 142, the display unit 143, the reading unit 144, RAM 145, the storage 146, I/F 147 and the bus 149, and thus the illustration thereof is omitted.

The control unit 341 has substantially the same construction and function as the control unit 141 of the portable terminal 140. Furthermore, it is preferable that the control unit 341 controls the input unit 342 described later to allow use of only a jog dial when the radiographing mode is set. Accordingly, there can be avoided such a situation that a radiographing operator inputs an erroneous key when radiography is carried out.

When the portable terminal 340 is set in the communication terminal 340a, the control unit 341 of the portable terminal 340 transmits/receives data to/from the control apparatus 360 which is connected to the communication terminal 340a through a cable.

When the portable terminal 340 is set in the communication terminal 340a after panel identification information is registered, the control unit 341 transmits the radiographing order information added with the panel identification information concerned through the communication terminal 340a to the control apparatus 360, and also deletes the transmitted data.

The control unit 341 rejects registration of the same panel identification information when the portable terminal 340 is carried out by a radiographing operator, that is, when the portable terminal 340 has not been set in the communication terminal 340a again to perform data transmission/reception to/from the control apparatus 360 after it was once carried by the operator.

The input unit 342 is designed to have cursor keys, numeric input keys, various function keys, etc. (all the elements are not shown), and it outputs an instruction signal input through the key operation of each key to the control unit 341.

The input unit 342 has a jog dial. The input unit 342 outputs to the control unit 341 an instruction signal to scroll (shift) each kind of display portion on the screen of the display unit 343 by dial operation of the jog dial, and also outputs an input (enter) instruction to the scrolled display portion to the control unit 341 by pushing the jog dial.

The input unit may have a touch panel equipped on the screen of the display unit 343. In this case, it outputs an instruction signal input through the touch panel.

The display unit 343, the reading unit 344, RAM 345 and the storage 346 have substantially the same constructions and functions as the display unit 143, the reading unit 144, RAM 145 and the storage unit 146 of the portable terminal 140 of the first embodiment, and thus the detailed description thereof is omitted.

I/F 347 is an interface adapted to RS-232C IrDA (Infrared Data Association) for transmitting/receiving data with the control apparatus 360 through the communication terminal 340a.

Here, the communication terminal 340a is connected to only one control apparatus through a cable in advance, and it has a communication control unit (not shown) for controlling data transmission/reception to/from the portable terminal 340 which is set in a throttle (not shown).

Next, the construction of the control apparatus 360 will be described.

As shown in FIG. 27, the control apparatus 360 is equipped with a control unit 361, an input unit 362, a display unit 363, a communication control unit 364, RAM 365, a storage 366, I/F 367 and an image processing unit 368, and these elements are connected to one another through a bus 369.

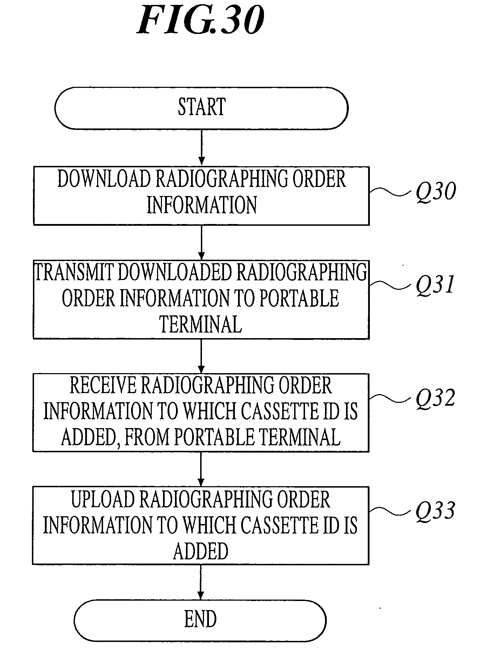

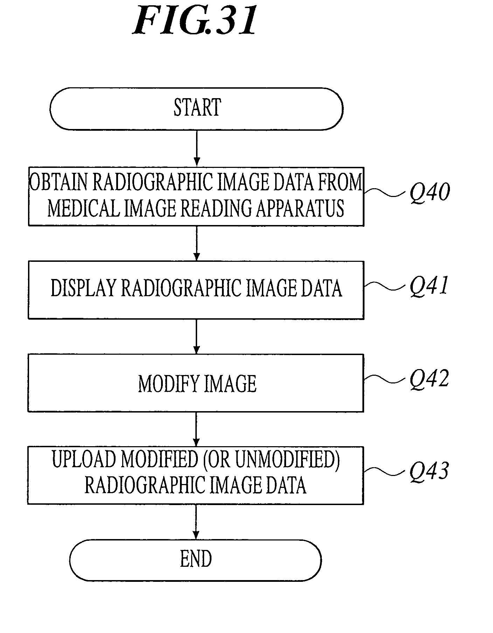

The control unit 361 reads out and executes various kinds of programs stored in the storage 365. Particularly, the control unit 361 reads out the programs for executing the communication processing, the image processing represented by the respective flowcharts of FIG. 30, FIG. 31 described in detail later, and executes the programs thus read out.

The control unit 361 selects a processing pattern (containing frequency processing, gradation processing, rotation processing, enlargement/reduction processing, etc.) matched with a radiographing condition, etc. described in the radiographing order information associated with radiographic image data which is achieved from the medical image reading apparatus 330, and makes the image processing unit 368 execute the image processing (containing the compression/expansion processing) based on the processing pattern concerned.

The control unit 361 stores the radiographic image data processed by the image processing unit 368 in association with the radiographing order information into the image DB 3172. Here, the processing pattern information matched with the radiographing condition is stored in the storage 365 in advance.

The input unit 362, the display unit 363 and RAM 364 have substantially the same constructions and functions as the control apparatuses 160a, 160b of the first embodiment, and thus the detailed description thereof is omitted.

The storage unit 365 is equipped with a non-volatile semiconductor memory such as HDD or the like, and stores various kinds of programs and various kinds of data. Particularly, the storage 365 stores the programs for executing the communication processing, the image processing represented by the respective flowcharts of FIG. 30, FIG. 31 described in detail later. The storage unit 365 stores plural processing patterns for executing the image processing matched with the radiographing conditions.

The storage 365 may have a recording medium (not shown) in which various kinds of programs and various kinds of data are stored in advance. The recording medium is a non-volatile memory which is fixedly or freely detachably equipped to the storage 365 and formed of a magnetic recording medium, an optical recording medium or a semiconductor memory.

The communication control unit 366 has substantially the same as the communication control unit 166 of the control apparatus 160a, 160b of the first embodiment, and thus the detailed description thereof is omitted.

I/F 367 is an interface adapted to RS-232C, IrDA, etc. to transmit/receive data to/from the portable terminal 340 through the communication terminal 340a.

Under the control of the control unit 361, the image processing unit 368 selects the processing pattern matched with the radiographing condition described in the radiographing order information associated with the radiographic image data achieved from the medical image reading apparatus 330, and executes the image processing (containing the compression/expansion processing) based on the processing pattern.

Next, the operation of the information management apparatus 310, the information portable 340 and the control apparatus 360 will be described with reference to FIGS. 28 to 31.

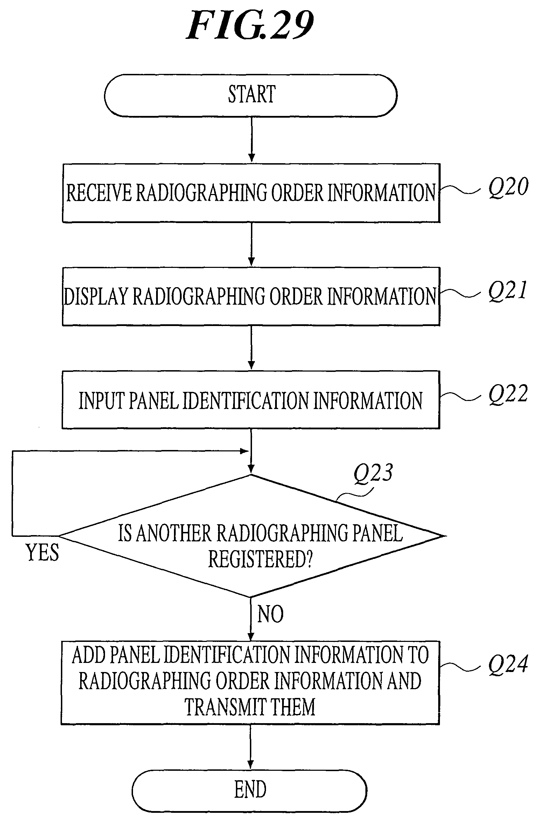

FIG. 28 is a flowchart showing the information processing carried out by the information management apparatus 310, FIG. 29 is a flowchart showing the communication processing carried out by the portable terminal 340, and FIGs. 30 and 31 are flowcharts showing the communication processing and the image processing carried out by the control apparatus 360, respectively.

First, the operation associated with the information processing of the information management apparatus 310 will be described.

Upon receiving new radiographing order information from HIS/RIS or the control apparatus 360 (step Q10), the control unit 311 records the new radiographing order information into a radiographing order information file 3171 (step Q11).

Upon receiving a signal for requesting radiographing order information from the control apparatus 360, the control unit 311 selects non-radiographed radiographing order information from radiographing order information stored in the radiographing order information file 3171, and transmits the non-radiographed radiographing order information thus selected to the control apparatus 360 serving as the request source concerned (step Q13). Here, the non-radiographed radiographing order information means radiographing order information added with no panel identification information.

When plural control apparatuses 360 are actuated and all these control apparatuses request radiographing order information, non-radiographed radiographing order information is transmitted to all the control apparatuses 360. The request for the radiographing order information is made by selectively making an input to a display portion B1 of a normal radiographing menu screen 3431a which is substantially the same as the normal radiographing menu screen 2431a shown in FIG. 14A or a display portion B3 of a portable radiographing menu screen 3431b which is substantially the same as the portable radiographing menu screen 2431b shown in FIG. 14B.

When radiographing order information for the portable radiography is particularly requested from the control apparatus 360 in step Q13, the control unit 311 selects non-radiographed radiographing order information for the portable radiography from the radiographing order information stored in the radiographing order information file 3171 while referring to portable indication flags which are added to the radiographing order information in advance, and transmits the radiographing order information thus selected to the control apparatus 360 serving as the request source concerned.

Upon receiving radiographing order information added with panel identification information from the control apparatus 360 (step Q14), the control unit 311 renews the radiographing order information concerned to the radiographing order information added with the panel identification information thus received (step Q15). Furthermore, when radiographing order information for renewal is transmitted from the control apparatus 360, the control unit 311 renews the corresponding radiographing order information to the radiographing order information for renewal.

Next, the operation associated with the communication processing of the portable terminal 340 will be described with reference to FIG. 29.

Upon receiving radiographing order information from the control apparatus 360 when the portable terminal 340 is set in the communication terminal 340a (step Q20), the control unit 341 displays the radiographing order information thus received on the screen of the display unit 343 (step Q21).

After the step Q21, when the portable terminal 340 is carried out by the radiographing operator and an operator's instruction is input through the input unit 342, the control unit 341 of the portable terminal 340 concerned operates in accordance with the input of the instruction concerned. For example, when the radiographing operator confirms that the patient information (patient's name, patient ID) of the radiographing order information displayed on the display unit 343 is coincident with the patient information of a patient to be actually radiographed and then inputs an instruction to read the panel identification information of a radiographing panel C used for radiography every radiographing condition displayed at the display portion A4, the control unit 341 directly reads the panel identification information (bar code information) of each radiographing panel from the radiographing panel concerned through the bar code reader of the reading unit 344 (step Q22, step Q23; Yes). At the display portion A5 of FIG. 328 are displayed as a radiographing condition both a radiographing condition (chest etc oblique) and the panel identification (bar code information) "04000108022016" of the radiographing panel for the radiography under the radiographing condition concerned which is read out at the stage of the step Q23. The above operation is repeated at the frequency corresponding to the number of radiographs to be achieved, and the radiographing condition and the radiographing panel are associated with each other in one-to-one correspondence.

In this case, the radiographing operator does not read out thepanel identification information of all the radiographing panels to be used for the radiography in a lump, but the operator preferably reads the panel identification information of each radiographing panel every time radiography is carried out, whereby there can be avoided such a situation that a radiographing panel is used under a radiographing condition different from the radiographing condition associated with the radiographing panel concerned.

When the portable terminal 340 is set in the communication terminal 340a and allowed to transmit/receive data to/from the control apparatus 360 after all the panel identification information to be used for radiography has been completely read out, that is, registration and radiography of the radiographing panels are finished (step Q23; No), the control unit 341 of the portable terminal 340 concerned adds the radiography-completed radiographing order information with the panel identification information concerned and transmits it to the control apparatus 360. In addition, the control unit 341 deletes the transmitted data (step Q24).

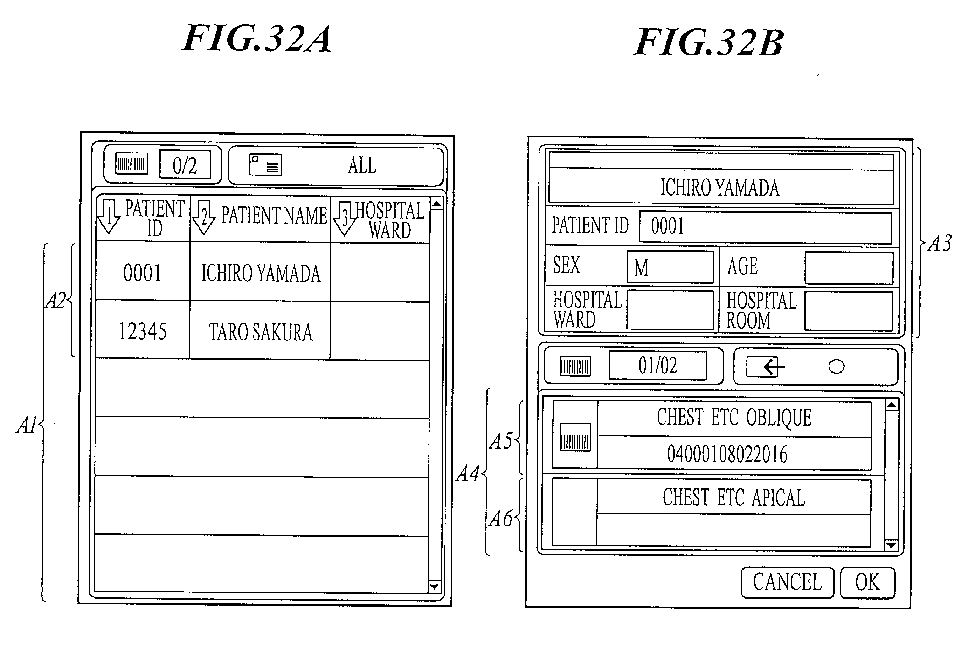

Here, FIG. 32A and FIG. 32B show display examples of the radiographing order information displayed on the screen of the display unit 343 at the step Q21. FIG. 32A shows a display example of patient's name and patient ID, and FIG. 32B shows a display example of a radiographing condition indicated every patient.

Patients' names and patent IDs are displayed in the form of a list at a display portion A1 on the screen shown in FIG. 32A, and particularly the patients' names and the patient IDs for two persons are displayed in the form of a list at a display portion A2 like the patient's name "Ichiro Yamada" and the patient ID "0001" are displayed while the patient's name "Taro Sakura" and the patient ID "12345" are displayed. The radiographing operator manipulates the jog dial of the input unit 342 to select the patient' s name and patient ID of any one of the two patients, and further pushes the jog dial under the above selection state to display the radiographing condition for the patient thus selected. For example, the screen shown in FIG. 32B is displayed as follows. That is, the radiographing operator selects the patient's name "Ichiro Yamada" and the patient ID "0001" by manipulating the jog dial at the display portion of the screen shown in FIG. 32A, and further pushing the jog dial under the selection state to display the radiographing condition for the patient of the patient's name "Ichiro Yamada" and the patient ID "0001" on the screen of the display unit 343.

On the screen shown in FIG. 32B, the patient' s name "Ichiro Yamada", the patient ID "0001",etc. are displayed at a display portion A3 and the radiographing condition "chest etc oblique", the panel identification information "04000108022016" (see a display portion A5), a radiographing condition "chest other Apical" (see a display portion A6), etc. are displayed in the form of a list at a display portion A4. The display portion A4 can be scrolled by the dial manipulation of the jog dial of the input unit 342, so that radiographing conditions which are protruded out of the display portion a4 can be displayed.

Next, the operation of the control apparatus 360 will be described.

The operation of the communication processing of the control apparatus 360 will be described with reference to FIG. 30.

When downloading radiographing order information from the information management apparatus 310 or makes a new input, the control unit 361 displays a normal radiographing menu screen 3431a substantially similar to the normal radiographing menu screen 2431a of FIG. 14A or a portable radiographing menu screen 3431b substantially similar to the portable radiographing menu screen 2431b of FIG. 14B in accordance with an input instruction of the radiographing operator on the screen of the display unit 363. In this case, when an instruction for indicating that all non-radiographed radiographing order information for normal radiography should be achieved is input through the input unit 362, the display on the normal radiographing menu screen 3431a is displayed on the screen of the display unit 363. When an instruction for indicating that all non-radiographed radiographing order information for portable radiography should be achieved is input through the input unit 362, the display on the portable radiographing menu screen 3431b is displayed on the screen of the display unit 363.

Here, when an input button "select operator" displayed at a display portion a1 is key-input through a mouse or touch panel of the input unit 362 on any screen of the normal radiographing menu screen 3431a and the portable radiographing menu screen 3431b, radiographing operator list information is transmitted from the information management apparatus 310, and a radiographing operation who intends to carry out radiography is allowed to be selected from the list.

When an input button "operation mode (normal)" displayed at a display portion a3 is key-input through the mouse or touch panel of the input unit 362 on the normal radiographing menu screen 3431a, the control unit 361 downloads all non-radiographed radiographing order information for normal radiography from the information management apparatus 310. When an input button "operation mode (portable)" displayed at a display portion a4 is key-input through the mouse or touch panel of the input unit 362 on the portable radiographing menu screen 3431b, the control unit 361 downloads all non-radiographed radiographing order information for portable radiography from the information management apparatus 310 (step Q30).

In this case, when no non-radiographed radiographing order information for portable radiography is registered in the information management apparatus 310, the radiographing operator inputs new radiographing order information for portable radiography or key-inputs the input button "operation mode (normal)" displayed at the display portion a3 through the mouse or touch panel of the input unit 362, downloads all non-radiographed radiographing order information for normal radiography from the information management apparatus 310, and alters some of the radiographing order information for normal radiography to radiographing order information for portable radiography as occasion demands. That is, when radiographing order information for normal radiography is downloaded, the radiographing operator selects radiographing order information altered so as to be adapted to portable radiography from the downloaded radiographing order information for normal radiography as occasion demands, and sets the portable indication flags of the selected radiographing order information to "'ON".

Returning to FIG. 30, the control unit 361 transmits the order information for portable radiography through the communication terminal 340a connected thereto to the portable terminal 340 set in the communication terminal 340a (step Q31). With respect to radiographing order information for portable radiography which are newly input and radiographing order information for portable radiography which are altered from the radiographing order information for normal radiography, the control unit 361 uploads these radiographing order information to the information management apparatus 310.

Upon receiving radiographing order information added with panel identification information, that is, radiographed radiographing order information from the portable terminal 340 set in the communication terminal 340a connected to the control apparatus 360 (step Q32), the control unit 361 of the control apparatus 360 uploads the radiographing order information added with the panel identification information thus received to the information management apparatus 310 (step Q33).

Next, the operation of the image processing of the control apparatus 360 will be described with reference to FIG. 31.

The control unit 361 achieves radiographic image data based on radiographing order information communicated between the control apparatus 360 of the control unit 361 concerned and the portable terminal 340 from the medical image reading apparatus 330 (step Q40). In this case, there are generally equippedmedical image reading apparatuses 330 of A, and the control unit 361 requests all the medical image reading apparatuses 330 of A to achieve the radiographic image data concerned.

At this time, in the medical image reading apparatus 330, it is checked whether radiographic image data associated with panel identification information coincident with each panel identification information transmitted from the control apparatus 360 are stored in a memory (not shown). If the radiographic image data whose panel identification information is coincident are stored in the memory, the radiographic image data are transmitted to the control apparatus 360 serving as the request source.

After the image processing of the image processing unit 368, the control unit 361 displays the radiographic image data achieved at the stage of the step Q40 on the screen of the display unit 363 together with the radiographing order information (step Q41).

After step Q41, the control unit 361 carries out image correction on the radiographic image data in accordance with an image correction instruction input through the input unit 362 (step Q42), and uploads the radiographic image data thus corrected to the information management apparatus 310 together with the radiographing order information (step Q43).

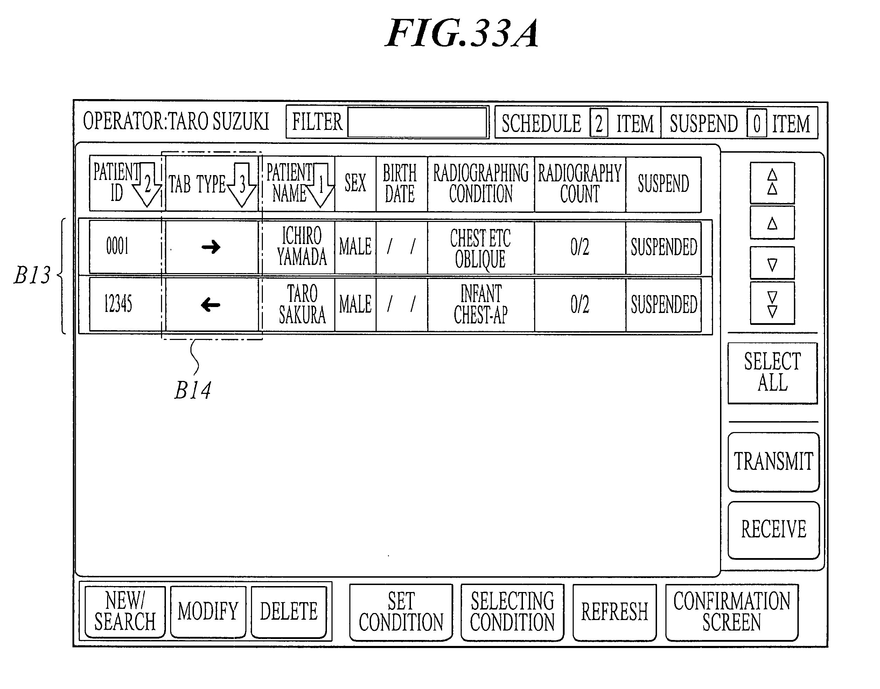

Next, the input operation of new radiographing order information in the control apparatus 360 will be described with reference to FIG. 17, FIG. 18 and FIG. 33A (see the description of the step Q31 and Q32 shown in FIG. 30) . The new radiographing order information is assumed to be indicated for portable radiography. Here, an input screen 3433 substantially similar to the input screen 2433 of the second embodiment shown in FIG. 17, a selection screen 3434 substantially similar to the selection screen 2434 shown in FIG. 18 and a screen of the display unit 363 shown in FIG. 33A are controlled by the control unit 361.

First, upon input of a registration instruction of new radiographing order information, the input screen 3433 substantially similar to the input screen 2433 shown in FIG. 17 is displayed on the screen of the display unit 363, and patient information such as patient's name and patient ID associated with the new radiographing order information is input through the input unit 362.

Input keys are displayed at a display portion c2 on the screen, and a key input of an input key is carried out through a mouse or touch panel of the input portion 362. The key input may be carried out through a keyboard equipped to the input unit 362. Input portion of patient ID, name (roman letter, Japanese (Kana characters, Chinese characters), English, etc.) are displayed at the display portion c1. When an input button "select operator" displayed at the display portion a1 of each of the normal radiographing menu screen 3431a and the portable radiographing menu screen 3431b is key-input through the mouse or touch panel of the input unit 362, the name of the radiographing operation thus selected is displayed at the display portion c3.

When the display portion c4 is key-input through the mouse or touch panel of the input unit 362, a selection screen which is substantially the same as the selection screen 2434 shown in FIG. 18 is displayed on the screen of the display unit 363, and the radiographing condition corresponding to the patient's name and the patient ID displayed at the input portion of the display portion c1 is set. When selection buttons for rough classification items "head, cervical part, .., TEST" of a radiographing condition are displayed at a display portion d2. When any one of the rough classification items of the radiographing condition concerned is key-input through the mouse or touch panel of the input unit 362, more detailed classification items "erect position of chest (A → P, .., side surface), .., observation of progress (P → A, infant PA)" for the input classification item (for example, chest) are displayed. For example when "chest etc oblique" and "chest etc Apical" in the classification items at the display portion d4 are key-input, the radiographing condition key-input at a display portion d5 is displayed as a list.

Here, the radiographing condition displayed as being hatched at the display portion d3 is a radiographing condition under which the portable radiography is impossible. As described above, the items of the radiographing condition under which the portable radiography is possible and the items of the radiographing condition under which the portable radiography is impossible are displayed in different display modes.

In this embodiment, the items of the portable-radiography-possible radiographing condition and the items of the portable-radiography-impossible radiographing condition are displayed in different display modes, however, the present invention is not limited to this display style. For example, the items of the portable-radiography-possible radiographing condition and the items of the portable-radiography-impossibleradiographingconditionmaybe displayed in the same display mode, and in this case when an erroneous key-input is carried out on the items of the portable-radiography-impossible radiographing condition, it may be warned with sounds or images that key-input to these items is prohibited.

When a key input is carried out on a display portion d6 through the mouse or touch panel of the input unit 362, a display shown in FIG. 33A is displayed on the screen of the display unit 363, and the patient's name, patient ID, etc. of the patient for whom the radiographing condition concerned is input are displayed in the form of a list. Information of two registered patients, that is, a patient of patient ID "0001" and patient's name "Ichiro Yamada" and a patient of patient ID "12345" and patient's name "Taro Sakura" is displayed in the form of a list.

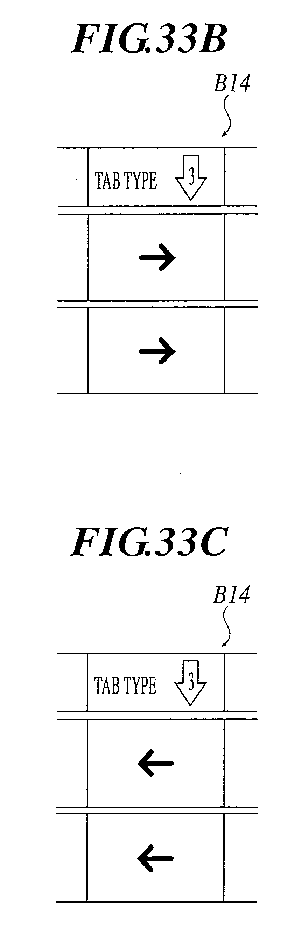

Here, when the radiographing order information of each patient in a display list like the display portion f1 is transmitted from the control apparatus 360 to the portable terminal 340, a symbol "→" (an arrow directing to the right) indicating this fact is displayed at the display portion f2 as shown in FIG. 33B. Furthermore, when radiographing order information is transmitted to the portable terminal 340 in accompany with panel identification information (that is, as radiographed radiographing order information), a symbol "←" (an arrow directing to the left) indicating this fact is displayed at the display portion f2 as shown in FIG. 33C. The symbols are not limited to "→" and "←", and other symbols may be displayed.

When an input button "CANCEL" displayed on the input screen 3433 substantially identical to the input screen 2433 shown in FIG. 17 and the selection screen 3434 substantially identical to the selection screen 2434 shown in FIG. 18 is key-input through the mouse or touch panel of the input unit 362, new radiographing order information in the input concerned is canceled by the control unit 361. Furthermore, even after the input of new radiographing order information is completed, when an input button "delete" displayed on the screen shown in FIG. 33 is key-input through the mouse or touch panel of the input unit 362, the input-completed radiographing order information concerned is deleted by the control unit 361.

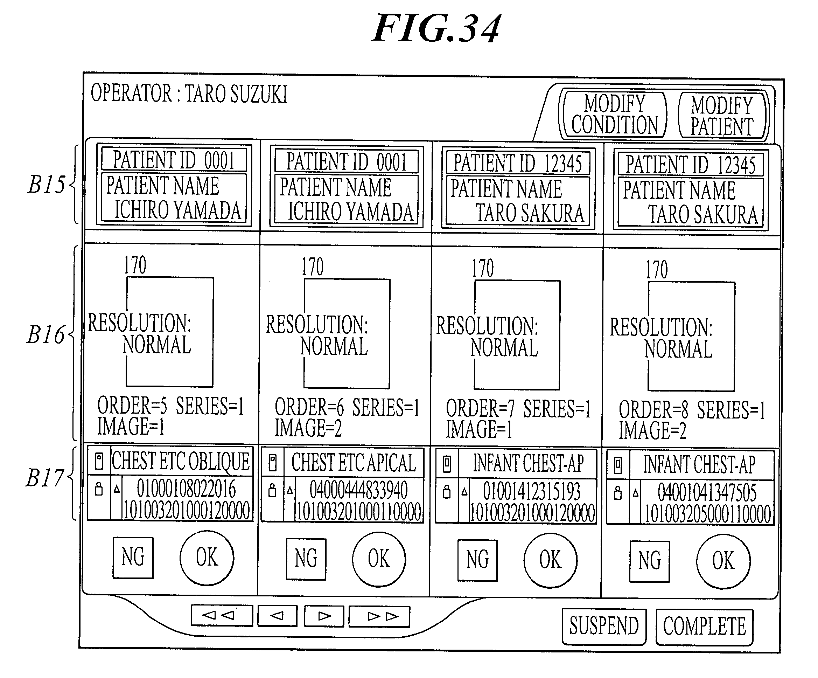

Next, the radiographic image displayed on the screen of the display unit 363 of the control apparatus 360 will be described with reference to FIG. 34 (see the description of the steps Q41 and Q42 of FIG. 31). The radiographic image data described hereunder is achieved by portable radiography. The display of the screen of the display unit 363 is controlled by the control unit 361.

First, when radiographing image data achieved by portable radiography are achieved from the medical image reading apparatus 330, a display as shown in FIG. 34 is displayed on the screen of the display unit 363. That is, the radiographic image data based on the portable radiography which are achieved from the medical image reading apparatus 330 correspond to radiographic images which have been processed by each portable terminal 340, and the radiographic images associated with plural radiographing conditions for each of plural patients, which have been processed by one portable terminal 340, are displayed in juxtaposition with one another. For example, two radiographic images associated with a patient (patient's name "Ichiro Yamada", patient ID "0001") and two radiographic images associated with a patient (patient' s name "Taro Sakura", patient ID "12345") are displayed at display portions B15 to B17 of FIG. 34. Radiographic images which protrude from one screen and thus cannot be displayed on the screen can be displayed by scrolling the screen.

Here, the patients ID and the patients' names are displayed at the display portion B15, radiographic images are displayed at the display portion B16, and the radiographing conditions, the identification numbers thereof and the panel identification information of panels used for radiography are displayed at the display portion B17.

As described above, the medical image photographing system 300 is equipped with the information management apparatus 310 for managing radiographing order information and radiographic image data, the medical image reading apparatus 330 for reading radiographic image data from radiographing panels C, theportable terminal 340 for associating radiographing order information with the panel identification information of a radiographing panel corresponding to each radiographing condition, the portable radiographing apparatus 350 for carrying out radiography (X-ray photography) at bet side in a hospital room, the control apparatus 360 for achieving radiographing order information from the information management apparatus 310, inputting new radiographing order information into the information management apparatus 310, and achieving and displaying radiographic image data from the medical image reading apparatus 330, etc.

One or plural communication terminals 340a are connected to each control apparatus 360 through a cable in advance, and each portable terminal 340 can transmit/receive data through the communication terminal 340a to/from only the control apparatus connected to the communication terminal 340.

Accordingly, even when the specifications of the portable terminal 340 and the control apparatus 3560 which are used at bed side are upgraded in version in order to expand the functions of the medical image photographing system 300, it is sufficient to merely change only the specifications of the control apparatus 360 and the portable terminal 340 connected to the control apparatus 360 through the communication terminal 340a, and it is unnecessary to change the overall medical image photographing system 300 and the specifications of other equipment such as the information management apparatus 310, the medical image reading apparatus 330, etc. Furthermore, in this case, it is unnecessary to change the specifications of the other portable terminals 340 and control apparatuses 360 which are not upgraded in version. Therefore, the version upgrade of the portable terminal 340 and the control apparatus 360, that is, the function expansion of the medical image photographing system 300 can be easily performed in low cost.

When a new portable radiographing system is introduced into facilities used for only normal radiography, it is sufficient to renew the control software for only a control apparatus 360 using a portable terminal(s) 340, and it is basically unnecessary to renew the other control apparatuses 360 and the network system at the upstream side of the control apparatus 360 concerned.

The foregoing description on this embodiment is made to an example of the medical image photographing system according to the present invention, and the present invention is not limited to this embodiment. The detailed construction and detailed operation of the medical image photographing system 300 according to this embodiment may be properly modified without departing from the subject matter of the present invention.

According to the present invention, even when the specifications of apparatuses used at bed side in a hospital room are upgraded in version in order to expand the functions of the medical image photographing system, it is unnecessary to change the overall medical image photographing system and the specifications of other apparatuses which are not used at bed side in a hospital room. Furthermore, in this case, it is unnecessary to change the specifications of other apparatuses which are used at bed side, but are not upgraded in version. Therefore, the version upgrade to expand the functions of the medical image photographing system can be easily performed in low cost.

That is, according to the present invention, the expansion of the functions of the medical image photographing system can be easily performed in low cost.

### [Fourth Embodiment]

The third embodiment relates to the medical image photographing system in which the portable terminal and the control apparatus are connected to each other in the master/slave connectionmode, and the radiographing condition and the cassette can be easily associated with each other by using the portable terminal in the master/slave connection mode in which the portable terminal can transmit/receive data to/from only the specific control apparatus. The fourth embodiment relates to a medical image photographing system in which radiographing order information transmitted from a control apparatus to a portable terminalisprohibitedfrom being transmitted to another portable terminal in a connection mode in which the communications between the portable terminal and the control apparatus are carried out in the master/slave connection mode.

The fourth embodiment according to the present invention will be described hereunder in detail with reference to the drawings.

In this embodiment, the medical image photographing system is equipped with plural portable terminals for displaying radiographing order information at a doctor' s round visit place. Furthermore, radiographing order information is transmitted from a control apparatus to a specific portable terminal to be displayed on the portable terminal, a flag indicating that radiographing order information has been already transmitted (hereinafter referred to as "transmission-completion flag") is set to radiographing order information transmitted froma control apparatus to some portable terminal, and the radiographing order information to which the transmission-completion flag is set is prohibited from being transmitted to another portable terminal.

First, the construction of this embodiment will be described.

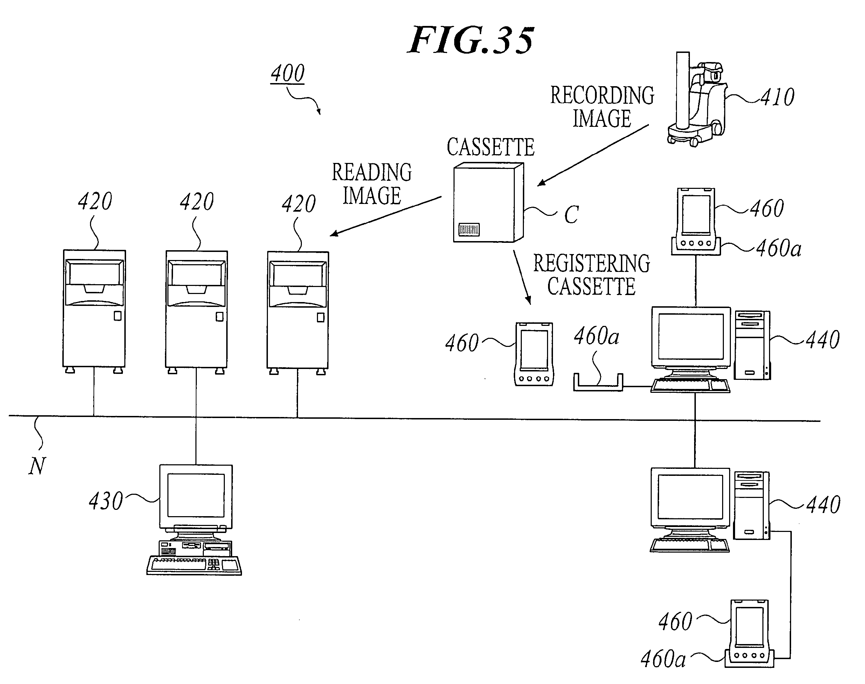

FIG. 35 shows the system construction of the medical image photographing system 400 according to this embodiment.

As shown in FIG. 35, the medical image photographing system 400 is equipped with a portable photographing apparatus 410, reading apparatuses 420 exclusively used for cassettes (cassette-dedicated reading apparatuses 420), an information management apparatus 430, control apparatuses 440, and portable terminals 460 substantially as in the case of the medical image photographing system 200 according to the second embodiment. The reading apparatuses 420, the information management apparatuses 430 and the control apparatuses 440 are connected to one another through a network N. A portable terminal 460 is connected to a specific control apparatus 440 through a communication network so as to transmit/receive information to/from the control apparatus 440. Each setup number of the control apparatuses 440 and the portable terminals 460 may be one or more, and it is not limited to a specific value.

The portable radiographing apparatus 410, the reading apparatus 420 and the information management apparatus 430 have substantially the same constructions and functions as the portable radiographing apparatus 210, the reading apparatus 220 and the information management apparatus 230 of the medical image photographing system of the second embodiment, and thus the detailed description thereof is omitted.

The control apparatus 440 controls the reading operation of medical images in the reading apparatus 420. It receives a medical image thus read out from the reading apparatus 420, and associates the medical image thus received with radiographing order information (hereinafter referred to as "image registration").

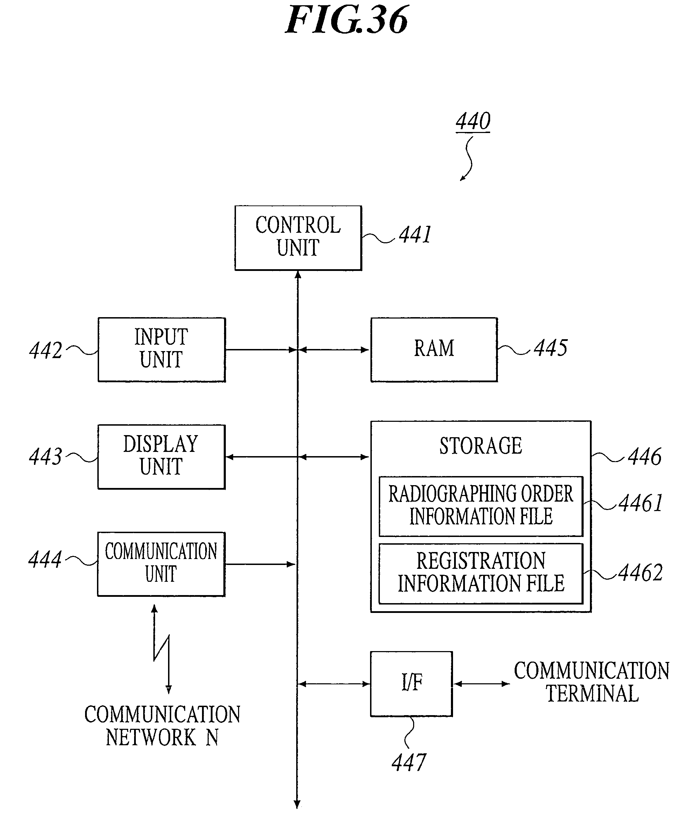

The functional construction of the control apparatus 440 is shown in FIG. 36.

As shown in FIG. 36, the control apparatus 440 is equipped with a control unit 41, an input unit 442, a display unit 443, a communication unit 444, RAM (Random Access Memory) 445, a storage 446, and I/F (InterFace) 447.

The control u nit 441, the input unit 442, the display unit 443, RAM 445 and the storage 446 have substantially the same constructions and functions as the control unit 241, the input unit 242, the display unit 243, RAM 245 and the storage 246 of the control apparatus 240 according to the second embodiment, and thus the detailed description thereof isomitted. A radiographing order information file 4461 and a registering information file 4462 contained in the storage 446 are substantially the same as the radiographing order information file 2461 shown in FIG. 10 and the registering information file 2462 shown in FIG. 11.

I/F 97 is an interface for connecting the control apparatus 440 and the communication terminal 460a, and outputs a detection signal to the control unit 441 when the portable terminal 460 is mounted in the communication terminal 460a. I/F 47 transmits radiographing order information to the portable terminal 460 through the communication terminal 460a, and receives cassette registering information for the transmitted radiographing order information concerned from the portable terminal 460.

Next, the portable terminal 460 will be described.

The portable terminal 460 is a portable type information processing device which can connect to a specific control apparatus 440 through the communication terminal 460a, and it receives radiographing order information based on portable radiography from the control apparatus 440 and displays it thereon. The portable terminal 460 associates radiographing order information with a cassette c used for the radiography based on the radiographing order information concerned (hereinafter referred to as "cassette registration"), and transmits the cassette registering information indicating the association result (correspondence relationship) to the control apparatus 440.

The communication terminal 460a is connected to a specific control apparatus 440 through a cable or the like, and controls transmission/reception of data between the portable terminal 460 mounted in the communication terminal 460a and the control apparatus 440. When the portable terminal 460 is mounted in the communication terminal 460a, the communication terminal 460a transmits a detection signal to the control apparatus 440, and charges the portable terminal 460 mounted therein.

The portable terminal 460 has substantially the same construction as the portable terminal 260 of the second embodiment shown in FIG. 12, and illustration of the portable terminal 460 is omitted.

Substantially like the portable terminal 260 shown in FIG. 12, the portable terminal 460 is equipped with a control unit 461, an input unit 462, a display unit 463, I/F 464, RAM 465, a storage 466 and a bar code reader 467.

The control unit 461, the input unit 462, the display unit 463, RAM 463 and the storage 465 have substantially the same constructions and functions as the control unit 261, the input unit 262, the display unit 263, RAM 265 and the storage 266 of the portable terminal 260 of the second embodiment respectively, and thus the detailed description thereof is omitted. A radiographing order information file 4661 and a cassette registering information file 4662 contained in the storage 466 are substantially the same as the radiographing order information file 2661 and the cassette registering information file 2662 of the second embodiment.

The bard code reader 4 67 is equipped with a scanner having an optical reading mechanism, and it reads out a bar code corresponding to a cassette ID from the surface of a cassette c and decodes the bar code according to a predetermined standard to achieve the cassette ID represented by the bar code. A patient ID may be affixed in the form of a bar code at patient's bed side or to a part of the patient's body, so that the patient ID is achieved by reading the affixed bar code with the bar code reader 467.

Next, the operation of this embodiment will be described.

First, the radiography preparation processing executed by the control apparatus 440 will be described. This radiography preparation processing is substantially identical to the radiography preparation processing executed by the control apparatus 240 of the medical image photographing system 200 of the second embodiment shown in FIG. 13 except for the order transmission processing of the step R9, and thus the description thereof is omitted except for the order transmission processing of the step R9.

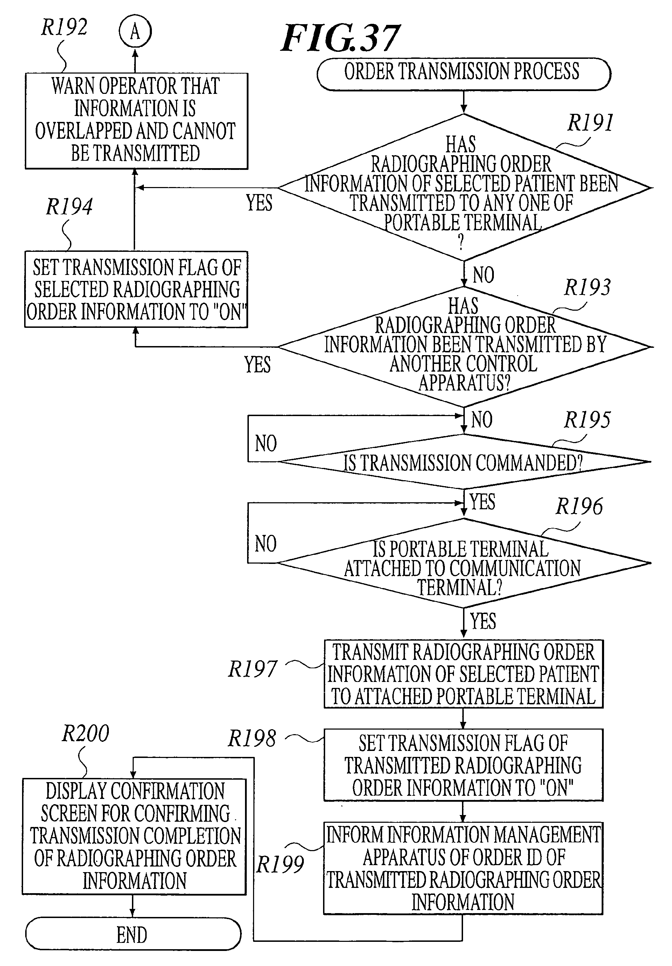

The order transmission processing of the step R9 will be described with reference to FIG. 37.

In the order transmission processing shown in FIG. 37, it is judged whether radiographing order information of a selected patient has been already transmitted to some portable terminal 460. This judgment is made on the basis of a judgement as to whether a transmission-completion flag is set to "ON" in the radiographing order information file 4461.

If the transmission-completion flag of the radiographing order information of the selected patient is set to "ON" and thus it is judged that the radiographing order information concerned has been transmitted to the portable terminal 460 (step R191; Y), a message warning that the radiographing order information concerned has been already transmitted to some portable terminal 460 and thus it cannot be redundantly transmitted is displayed on the display unit 443, or a sound warning this fact is output (step R192) . After the warning, the processing goes to substantially the same processing as the step R8 of FIG. 13.

On the other hand, if the transmission-completion flag of the radiographing order information of the selected patient is set to "OFF" and thus it is judged that the radiographing order information concerned has not yet been transmitted to any portable terminal 460 (step R191; N), the order ID of the radiographing order information of the selected patient is transmitted to the information management apparatus 430, and inquiry information for inquiring about whether the radiographing order information concerned has been transmitted from another control apparatus 440 to some portable terminal 460 is transmitted to the information management apparatus 430. On the basis of response information received from the information management apparatus 430, it is judged whether the radiographing order information of the selected patient has been already transmitted from another control apparatus 440 to some portable terminal 460 (step R193).

If it is judged that the radiographing order information of the selected patient has been transmitted from another control apparatus 440 to some portable terminal 460 (step R193; Y) , the transmission-completion flag of the radiographing order information of the selected patient is set to "ON" in the radiographing order information file 4461 (step R194), and then the processing goes to step R192 to warn that the radiographing order information concerned has been already transmitted to some portable terminal 460 and thus it cannot be redundantly transmitted. After the warning, the processing goes to substantially the same processing as the step R8 of FIG. 13.

On the other hand, when the selected radiographing order has not yet been transmitted (step R193; N), an input of a transmission instruction by the transmission key b3 is awaited, and it is judged in step R195 whether the transmission input is input or not. If the transmission instruction is input (step R195; Y) , on the basis of a detection signal of a portable terminal 460 input from the communication terminal 460a, it is judged whether some portable terminal 460 is mounted in the communication terminal 460a (step R196).

If no detection signal is output and thus it is judged that no portable terminal 460 is mounted in the communication terminal 460 (step R196; N), the processing waits until a portable terminal 460 is mounted. If the detection signal is output and thus it is judged that some portable terminal 460 is mounted in the communication terminal 460a (step R196; Y), the radiographing order information of the selected patient is transmitted to the portable terminal 460 mounted in the communication terminal 460a by the communication unit 444 (step R197). After the transmission is finished, the transmission-completion flag of the radiographing order information transmitted to the portable terminal 460 is set to "ON" in the radiographing order information file 4461 in step R198.

Subsequently, in step R199, the order ID of the radiographing order information for which the transmission-completion flag is set to "ON" is transmitted to the information management apparatus 430, and it is notified that the radiographing order information has been transmitted. In the information management apparatus 430, the radiographing order information of the order ID transmitted from the control apparatus 440 is managed as transmission-completion radiographing information.

Subsequently, in step R200, a check screen 4435 (substantially identical to the check screen 2435 shown in FIG. 20A) for checking transmission-completion of radiographing order information is displayed on the display unit 443, and then this processing is finished. In the check screen 4435 substantially identical to the check screen 2435 shown in FIG. 20A, atransmission/reception status b5 isequippedeverypatient of a display list, and a mark "→" representing transmission-completion as in the case of FIG. 20B is displayed at the transmission/reception status b5 of each patient whose radiographing order information is transmitted to the portable terminal 460 in the list-displayed patients. Furthermore, when cassette registering information is received from the portable terminal 460, a mark "←" representing reception-completion as in the case of FIG. 20C is displayedat the transmission/reception status b5 of the patient.

When the radiographing operator confirms that the radiographing order information is transmitted to the portable terminal 460 on the check screen 4435, the operator carries out the cassette c and the portable radiographing apparatus 410 to the patient to be radiographed while carrying the portable terminal 460.

Subsequently, the cassette registration processing executed by the portable terminal 460 will be described. This cassette registration processing is substantially identical to the cassette registration processing executed by the portable terminal 260 of the medical image photographing system of the second embodiment shown in FIG. 21 except for the processing of the step P8 and the subsequent steps, and thus the description thereof is omitted.

In step P8, it is judged whether the portable terminal 460 is mounted in the communication terminal 460a or not. If the portable terminal 460 is not mounted in the communication terminal 460a (step P8; N), the processing waits until the portable terminal 460 is mounted. If the portable terminal 460 is mounted in the communication terminal 460a (step P8; Y), cassette registering information is transmitted through the communication terminal 460a to the control apparatus 440 connected to the communication terminal 460 concerned (step P9) , and then this processing is finished.

After the radiographing operator sets the portable terminal 460 in the communication terminal 460a and transmits the cassette registering information to the control apparatus 440, the operator sets the cassette c in the reading apparatus 420, and operates the control apparatus 440 to instruct reading of a medical image recorded in the cassette c. In the reading apparatus420, thebardcodeofthecassetteIDisreadouttogether with the medical image from the cassette c thus set to achieve the cassette ID, and the cassette ID thus read out is written into the header area of the read-out medical image. The medical image in which the cassette ID is written is transmitted to the control apparatus 440.

The image registration processing executed by the control apparatus 440 is substantially identical to the image registration processing executed by the control apparatus 240 of the medical image photographing system 200 according to the second embodiment shown in FIG. 23, and thus the description thereof is omitted.

As described above, in the medical image photographing system 400, plural portable terminals 460 are connected to the communication network, and the radiographing order information based on portable radiography is transmitted from the control apparatus 440 to the portable terminal 460 to be displayed on the portable terminal 460. Therefore, even when radiography is carried out at a doctor's round visit place such as a bed side of a patient or the like, a radiographing operator can check radiographing information through a portable terminal 460 carried by the operator, and thus the operator can easily check the patient to be radiographed and the radiographing condition for the radiography. Accordingly, the radiography to achieve medical image at a doctor' s round visit place can be efficiently and accurately performed.

Furthermore, when ID of a cassette used for radiography is read out in the portable terminal 460 to carry out cassette registration and then the cassette registering information is transmitted from the portable terminal 460 to the control apparatus 440, the medical image achieved through the radiography and the radiographing order information are associated with each other on the basis of the cassette registering information in the control apparatus 440. Therefore, the operator's work of manually inputting the correspondence relationship between the radiographing order information and the medical image into the control apparatus 440 can be omitted, and thus the working load imposed on the operator can be reduced. Furthermore, a human error caused by an erroneous input of a radiographing operator can be prevented, and medical images can be accurately managed.

Furthermore, the transmission-completion flag of the radiographing order information transmitted from a control apparatus 440 to any one of the portable terminals 460 is set to "ON", and thus the radiographing order information for which the transmission-completion flag is set to "ON" is prohibited from being transmitted to another portable terminal 460, so that the same radiographing order information can be prevented from being redundantly transmitted to plural portable terminals.

Still furthermore, in the information management apparatus 430, the transmission status of radiographing order information which is simultaneously transmitted to plural control apparatuses 440 is managed, and radiographing order information which has been already transmitted from a control apparatus 440 to any one of portable terminals 460 is prohibited from being transmitted from another control apparatus 440 to any one of the portable terminals 460, so that the same radiographing order information can be prevented from being redundantly transmitted to plural portable terminals.

Still furthermore, when radiographing order information for which the transmission-completion flag is set to "ON" is indicated as radiographing order information to be transmitted to a portable terminal 460 by a radiographing operator, it is warned that it is impossible to transmit the radiographing order information concerned because it has been already transmitted. Therefore, operator's attention can be attracted to the fact that the radiographing order information concerned has been already transmitted (i.e., transmission has been completed), and the radiographing order information can be prevented from being redundantly transmitted by mistake.

As described above, by preventing redundant transmission of radiographing order information, plural radiographing operators can be prevented from simultaneously preparing for radiography for the same patient, or radiography can be prevented from being redundantly carried out on the basis of the same radiographing order information. Therefore, radiography to achieve medical images at a doctor's round visit place can be accurately performed.

In the system construction described above, a portable terminal 460 is directly connected to a predetermined control apparatus 440 through the communication terminal 460a. Therefore, even when system maintenance is carried out or the system is changed, it is sufficient to carry out maintenance on only a specific control apparatus 440 and a portable terminal 460 connected to the control apparatus 440, so that the degree of freedom of system change can be enhanced and the re-construction of the system can be facilitated. Furthermore, the system can be stabilized by specifying a control apparatus 440 to which a portable terminal is connected.

The description content of the above embodiment is made to a preferable embodiment of the medical image photographing system 400 to which the present invention is applied, and thus the present invention is not limited to this embodiment.

For example, in the foregoing description, when radiographing order information indicated by the portable terminal 460 is transmitted from the control apparatus 440, it is inquired to the information management apparatus 430 whether the radiographing order information concerned has been already transmitted from another control apparatus. However, the present invention is not limited to this embodiment. For example, when it is notified from each control apparatus 440 to the information management apparatus 430 that some radiographing order information has been already transmitted, the order ID of the transmitted radiographing order information concerned is distributed to each control apparatus 440 to notify that the radiographing order information has been already transmitted from another control apparatus, whereby the transmission status of radiographing order information can be grasped on a real-time basis at each control apparatus 440.

Besides, the detailed construction and operation of the medical image photographing system 400 may be properly modified without departing from the subject matter of the present invention.

According to the present invention, since radiographing order information is displayed on a portable terminal, a radiographing operator can easily check a patient to be radiographed and a radiographing condition by using the portable terminal even when the operator carries out radiography at a doctor's round visit place such as the bet side of the patient or the like. Accordingly, the radiography at the doctor's round visit place can be efficiently performed. Furthermore, radiographing order information which has been transmitted from a control apparatus to a portable terminal is prohibited from being transmitted to another portable terminal, so that the same radiographing order information can be prevented from being redundantly transmitted to plural portable terminals. Accordingly, redundant radiography on the same patient can be prevented, and the radiography at a doctor's round visit place can be accurately performed.

Still furthermore, radiographing order information which has been transmitted from a control apparatus to any portable terminal is prohibited from being transmitted from another control apparatus, so that the same radiographing order information can be prevented from being redundantly transmitted to plural portable terminals. Accordingly, redundant radiography based on the same radiographing order information can be prevented, and the radiography at a doctor' s round visit place can be accurately performed.

Still furthermore, it is warned that transmission of radiographing order information to some portable terminal has been completed, so that operator's attention can be attracted to the fact that the radiographing order information has been already transmitted.

The entire disclosure of Japanese Patent Applications No. Tokugan 2002-317243 filed on October 31, 2002, No. 2003-084708 filed on March 26, 2003, No. 2003-084753 filed on March 26, 2003 and No. 2003-091600 filed on March 28, 2003 including specification, claims drawings and summary are incorporated herein by reference in its entirety.

## Claims

1. A medical image photographing system comprising:
a medical image generating apparatus for photographing a patient to generate image data of the patient;
an information management apparatus for generating photographing order information including patient identification information of the patient to be photographed and/or a photographing condition for the patient, the information management apparatus being allowed to transmit the generated photographing order information through a network to another apparatus;
one or more control apparatuses that are connectable to the information management apparatus through the network to obtain the generated photographing order information; and
one or more portable terminals that are connectable to at least one of the control apparatuses to obtain the photographing order information from the control apparatus connected thereto and display the obtained photographing order information.

2. The medical image photographing system of claim 1, further comprising a plurality of control apparatuses,
wherein each of the one or more portable terminals is connectable to all of the plurality of control apparatuses through the network.

3. The medical image photographing system of claim 2, wherein the medical image generating apparatus uses an image recording panel whenever the patient is photographed;
the one or more portable terminals obtain recording panel identification information of each image recording panel, and store the photographing order information and the obtained recording panel identification information so as to set a correspondence of the photographing order information to the obtained recording panel identification information;
the one or more portable terminals transmits the stored photographing order information and the stored recording panel identification information to one of the plurality of control apparatuses;
the one of the plurality of control apparatuses transmits the photographing order information and the recording panel identification information received from the one ormore portable terminals, to the information management apparatus; and
the information management apparatus stores the recording panel identification information so as to correspond to the photographing order information.

4. The medical image photographing system of claim 3, further comprising an image information reading apparatus for reading out a photographing image from the image recording panel,
wherein the image information reading apparatus reads out the recording panel identification information of the image information recording panel, obtains relating information of the photographing orderinformation,whichincludesa processing condition through the control apparatus, and reads out the image under the obtained processing condition.

5. The medical image photographing system of claim 2, further comprising a plurality of portable terminals,
wherein the information management apparatus stores identification information of a first portable terminal of the plurality portable terminals for reading out the photographing order information, so as to correspond to the read out photographing order information; and when the photographing order information is read out by the first portable terminal of the plurality of portable terminals and then a command for reading out the photographing order information is output from a second portable terminal which is different from the first portable terminal of the plurality of portable terminals, the second portable terminal of the plurality of portable terminals is notified that the photographing order information has been already read out on the basis of the stored information.

6. The medical image photographing system of claim 2, further comprising a plurality of portable terminals,
wherein the photographing order information, transmitted from one of the plurality of control apparatuses to one of the plurality of portable terminals, is prohibited from being transmitted from the control apparatus to another portable terminal.

7. The medical image photographing system of claim 2, wherein the photographing order information, transmitted from one of the plurality of control apparatuses to any one of the one or more portable terminals, is prohibited from being transmitted from another control apparatus to any one of the one or more portable terminals.

8. The medical image photographing system of claim 6, wherein when each of the plurality of control apparatuses is instructed so that the photographing order information transmitted to any one of the plurality of portable terminals is transmitted to any one of the plurality of portable terminals again, the control apparatus warns that the photographing order information has been already transmitted.

9. The medical image photographing system of claim 6, wherein after the photographing order information is transmitted from one of the plurality of control apparatuses to any one of the plurality of portable terminals, another control apparatus deletes the photographing order information.

10. The medical image photographing system of claim 1, further comprising a plurality of control apparatuses,
wherein each of the one or more portable terminals is connectable to a specific control apparatus of the plurality of control apparatuses.

11. The medical image photographing system of claim 10, wherein the medical image generating apparatus uses an image recording panel whenever the patient is photographed;
the one or more portable terminals obtain recording panel identification information of each image recording panel, and store the photographing order information and the obtained recording panel identification information so as to set a correspondence of the photographing order information to the obtained recording panel identification information;
the one or more portable terminals transmits the stored photographing order information and the stored recording panel identification information to the specific control apparatuses;
the specific control apparatus transmits the photographing order information and the recording panel identificationinformationreceivedfrom the one or more portable terminals, to the information management apparatus; and
the information management apparatus stores the recording panel identification information so as to correspond to the photographing order information.

12. The medical image photographing system of claim 11, further comprising an image information reading apparatus for reading out a photographing image from the image recording panel,
wherein the image information reading apparatus reads out the recording panel identification information of the image information recording panel, obtains relating information of the photographing order information, which includes a processing condition through a control apparatus, and reads out the image under the obtained processing condition.

13. The medical image photographing system of claim 11, wherein when the photographing order information corresponding to the panel identification information is transmitted to the specific control apparatus, each of the one or more portable terminals transmits the photographing order information while adding information indicating that the photograph is finished, to the photographing order information.

14. The medical image photographing system of claim 10, wherein each of the one or more portable terminals collates input patient identification information with patient identification information including the photographing order information, and notifies a collation result.

15. The medical image photographing system of claim 10, further comprising a plurality of portable terminals,
wherein the photographing order information, transmitted from one of the plurality of control apparatuses to one of the plurality of portable terminals, is prohibited from being transmitted to another portable terminal.

16. The medical image photographing system of claim 15, wherein the photographing order information, transmitted from one of the plurality of control apparatuses to any one of the plurality of portable terminals, is prohibited from being transmitted from another control apparatus to any one of the plurality of portable terminals.

17. The medical image photographing system of claim 15, wherein when each of the plurality of control apparatuses is instructed so that the photographing order information transmitted to any one of the plurality of portable terminals is selected to be transmitted to any one of the plurality of portable terminals again, the control apparatus warns that the photographing order information has been already transmitted.

18. The medical image photographing system of claim 15, wherein after the photographing order information is transmitted from one of the plurality of control apparatuses to any one of the plurality of portable terminals, another control apparatus deletes the photographing order information.

19. A medical image photographing method in a medical image photographing system comprising an information management apparatus, one or more control apparatuses and one or more portable terminals, comprising:
photographing a patient to generate image data of the patient;
generating photographing order information including patient identification information of the patient to be photographed and/or a photographing condition for the patient;
transmitting the generated photographing order information through a network to another apparatus;
obtaining the transmitted photographing order information by transmitting the transmitted photographing order information from the control apparatus to the portable terminal;
display the obtained photographing order information.

20. The medical image photographing method of claim 19, wherein the medical image photographing system further comprises a plurality of control apparatuses,
wherein the medical image photographing method further comprises:
using an image recording panel whenever the patient is photographed;
obtaining recording panel identification information of each image recording panel;
storing the photographing order information and the obtained recording panel identification information so as to set a correspondence of the photographing order information to the obtained recording panel identification information;
transmitting the stored photographing order information and the stored recording panel identification information to one of the plurality of control apparatuses;
transmitting the photographing order information and the recording panel identification information received from the one or more portable terminals, to the information management apparatus; and
storing the recording panel identification information in the information management apparatus so as to correspond to the photographing order information.

21. The medical image photographing method of claim 20, further comprising:
obtains relating information of the photographing order information, which includes a processing condition through the control apparatus, and
reading out the image under the obtained processing condition.

22. The medical image photographing method of claim 19, wherein the medical image photographing system further comprises a plurality of portable terminals; and
wherein the medical image photographing method further comprises:
storing identification information of a first portable terminal of the plurality portable terminals for reading out the photographing order information, so as to correspond to the read out photographing order information;
wherein when the photographing order information is read out by the first portable terminal of the plurality of portable terminals and then a command for reading out the photographing order information is output from a second portable terminal which is different from the first portable terminal of the plurality of portable terminals, the second portable terminal of the plurality of portable terminals is notified that the photographing order information has been already read out on the basis of the stored information.

23. The medical image photographing method of claim 19, wherein the medical image photographing system further comprises a plurality of portable terminals; and
wherein the photographing order information, transmitted from one of the plurality of control apparatuses to one of the plurality of portable terminals, is prohibited from being transmitted from the control apparatus to another portable terminal.

24. The medical image photographing method of claim 23, further comprising:
prohibiting the photographing order information transmitted from one of the control apparatuses to any one of the one or more portable terminals, from being transmitted from another control apparatus to any one of the one or more portable terminals.

25. The medical image photographing method of claim 19, wherein when each of the plurality of control apparatuses is instructed so that the photographing order information transmitted to any one of the plurality of portable terminals is transmitted to any one of the plurality of portable terminals again, the control apparatus warns that the photographing order information has been already transmitted.

26. The medical image photographing method of claim 19, wherein afterthe photographing orderinformationistransmitted from one of the control apparatuses to any one of the plurality of portable terminals, another control apparatus deletes the photographing order information.

27. The medical image photographing method of claim 20, wherein when the photographing order information corresponding to the panel identification information is transmitted to a specific control apparatus, each of the one or more portable terminals transmits the photographing order information while adding information indicating that the photograph is finished, to the photographing order information.

28. The medical image photographing method of claim 19, further comprising:
inputting patient identification information to the portable terminal;
collating input patient identification information with patient identification information including the photographing order information; and
notifying a collation result.
